# EUROPEAN PATENT APPLICATION

(11) **EP 4 417 203 A1**
(43) Date of publication of application: **21.08.2024**
(21) Application number: 24157039.9
(22) Date of filing: 12.02.2024
(51) Int. Cl.: A61K 31/437, A61K 45/06, A61P 35/00

(54) **SELECTIVE ANTI-CANCER ACTION OF DISTINCT IMIDAZOPYRIDINE COMPOUNDS IN STEROID HORMONE RECEPTOR-POSITIVE BREAST, PROSTATE, AND ENDOMETIRAL CANCER**

(30) Priority: 16.02.2023 EP 23156969
(71) Applicant: Karlsruher Institut für Technologie, 76131 Karlsruhe (DE)
(72) Inventor: Cato, Andrew C. B, 76344 Eggenstein-Leopoldshafen (DE); Pan, Mengwu, 76185 Karlsruhe (DE); Solozobova, Valeria, 75045 Walzbachtal (DE); Kuznik, Nane, 79709 Kronau (DE); Stober, Jutta, 76661 Philippsburg (DE); Gräßle, Simone, 76571 Gaggenau (DE); Jung, Nicole, 68163 Mannheim (DE); Bräse, Stefan, 53842 Troisdorf (DE)
(74) Representative: Müller-Boré & Partner Patentanwälte PartG mbB

(57) **Abstract**

The present invention relates to specific imidazopyridine compounds for use in the treatment of breast cancers, prostate cancers, and endometrial cancers.

## Description

The present invention relates to specific imidazopyridine compounds for use in the treatment of breast cancers, prostate cancers, and endometrial cancers.

Breast cancer (BCa) and prostate cancer (PCa) are initially both steroid hormone-dependent cancers that can be treated by hormone therapy. The steroid hormones androgen and estrogen function by binding to their respective receptors (androgen receptor (AR) and estrogen receptor α (ERα)) in prostate and breast cancer cells to enhance proliferation of the tumor cells. Almost all prostate cancers begin in an androgen-dependent state, where AR signaling is predominant for cancerous growth and proliferation. Similarly, ERα is overexpressed in approximately 70% of breast cancers. Targeted endocrine therapies are therefore given to prevent primary prostate and breast cancer metastasis. These therapeutic paradigms significantly reduce the 5 years risk of recurrence and continually show improved disease outcomes. Afterwards, resistance to the endocrine therapies develop and these remain a major source of cancer mortality. Approximately 30% to 50% of patients present acquired or *de novo* resistance to endocrine therapies after an average of 2 to 5 years.

There is therefore a pressing need to develop next generation antagonists that can effectively suppress the endocrine resistance. In prostate cancer (PCa), the current therapies aimed at reducing AR signaling all target the C-terminal ligand-binding domain (LBD) of the receptor. Although these therapies are initially successful, patients invariably also become resistant to treatment and develop a more aggressive form of the disease termed castration resistant PCa (CRPC) which, in most cases, remains dependent on AR signaling for growth.

Thus, therapies that target other domains of the AR or other regulatory factors are also needed.

In both BCa and PCa, one of the major reasons for the resistance to endocrine therapy is the occurrence of mutations in the ligand binding domain (LBD) of the receptors and these mutations obviate the action of the hormone antagonists. Therefore, the development of new antagonists of ERα and AR that do not function through the LBDs of these receptors but rather function indirectly through cofactors that enhance the activities of the receptors is needed.

In addition to BCa, signaling by ERα and its ligand 17 β-estradiol (E₂) is closely associated with the occurrence and development of endometrial carcinoma (EC), one of the most common carcinomas of the female reproductive system. EC can be divided into two types: ERα / E₂-dependent EC (type I) and non-E₂-dependent EC (type II). The majority of type I ECs are endometrioid carcinomas, which are well differentiated, and a few are mucinous adenocarcinomas. Type II ECs include serous carcinoma and clear cell carcinoma. The majority of type II ECs do not express ERα and may develop in a hormone-independent manner. In addition to the wild-type ERα, some mutations and splice variants of ERα mRNA and ERα protein have been shown to influence the occurrence and development of EC. As opposed to BCa where ERα is the most common target for therapy, anti-ERα therapy with classical ER antagonists such as tamoxifen has shown inconsistent results in EC, with very limited therapeutic efficacy and sometimes even an increased risk of cancer possibly due to the ER mutants and variants. There is therefore the need to identify new and improved ERα-targeting drugs for EC.

Accordingly, the technical problem underlying the present invention is the provision of such antagonists, *i.e.,* antagonists of ERα and AR that do not function through the LBDs of these receptors but rather function indirectly through cofactors that enhance the activities of the receptors, which can be used in the treatment of breast cancer, prostate cancer, and endometrial cancer.

The solution to the above technical problem is achieved by the embodiments characterized in the claims.

In particular, the present invention relates to a compound for use in the treatment of cancer in a subject, wherein the cancer is selected from the group consisting of breast cancer, prostate cancer, and endometrial cancer, wherein said compound is a compound according to Formula (I): 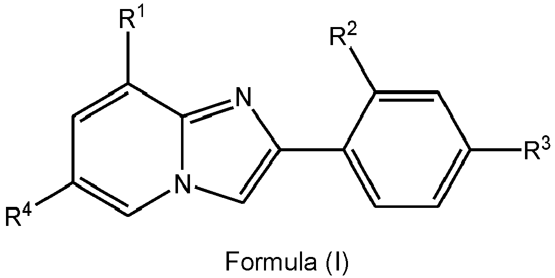 wherein
R¹ is H or Cl,
R² is H or F,
R³ is H or F, and
R⁴ is CH₃ or CF₃.

The compounds for use according to the present invention are imidazopyridine compounds according to Formula (I) as defined above. In preferred embodiments, the compound according to Formula (I) is a compound, selected from the group consisting of the following compounds X15695, X15696 (=X19166), X19720, X19724, X19728, and X20046: 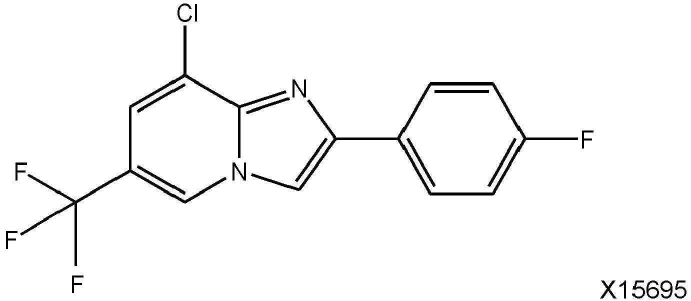 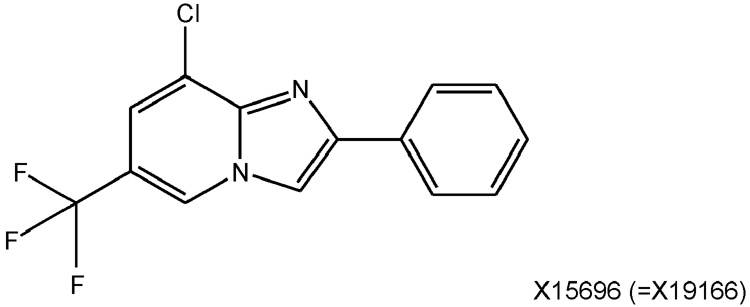 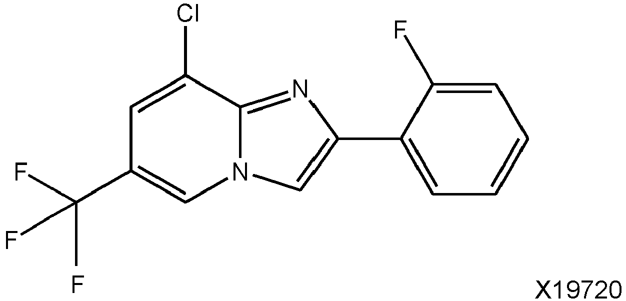 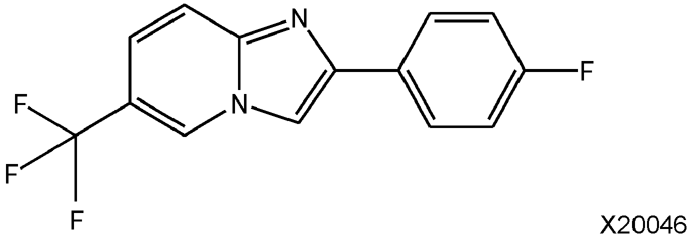 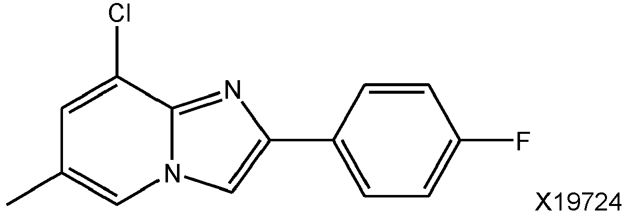 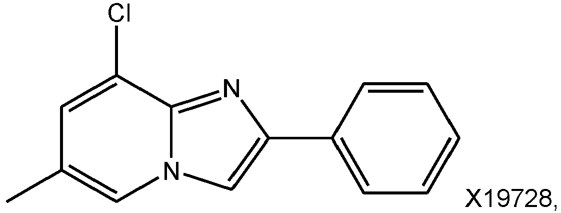 wherein the compound X15695 is particularly preferred.

IUPAC names for the above compounds are as follows:
X15695:
   8-Chloro-2-(4-fluorophenyl)-6-(trifluoromethyl)imidazo[1,2-a]pyridine
X15696 (also designated X19166):
   8-Chloro-2-phenyl-6-(trifluoromethyl)imidazo[1,2-a]pyridine
X19720:
   8-Chloro-2-(2-fluorophenyl)-6-(trifluoromethyl)imidazo[1,2-a]pyridine
X19724:
   8-Chloro-2-(4-fluorophenyl)-6-methylimidazo[1,2-a]pyridine
X19728:
   8-Chloro-6-methyl-2-phenylimidazo[1,2-a]pyridine
X20046:
   2-(4-Fluorophenyl)-6-(trifluoromethyl)imidazo[1,2-a]pyridine

The cancers to be treated according to the present invention are breast cancer, prostate cancer, and endometrial cancer, i.e., cancers with deregulated expression of estrogen and/or androgen receptors. In this context, deregulated expression of estrogen and/or androgen receptors preferably comprises overexpression of estrogen and/or androgen receptors, expression of estrogen and/or androgen receptors having point mutations, expression of estrogen and/or androgen receptors having a deletion, and/or expression of splice variants of estrogen and/or androgen receptors. Respective cancers, as well as respective mutated forms of estrogen and/or androgen receptors (concerning point mutations as well as deletions), and respective splice variants of estrogen and/or androgen receptors are known in the art.

In specific embodiments, the estrogen receptor is estrogen receptor α (ER α) and/or the androgen receptor is androgen receptor (AR).

In preferred embodiments, the cancer to be treated according to the present invention is breast cancer, preferably estrogen receptor-positive breast cancer, e.g. ER α-positive breast cancer. In specific examples, the breast cancer displays positive expression of progesterone receptor (PR) and/or human epidermal growth factor receptor 2 (HER2), e.g., luminal A (ER⁺, PR⁺, HER2-), luminal B (ER⁺, PR⁻, HER2⁺) or luminal B-like (ER⁺, PR⁺, HER2⁺) breast cancer.

In other preferred embodiments, the cancer to be treated according to the present invention is prostate cancer, preferably androgen receptor-positive prostate cancer, e.g. castration-resistant prostate cancer.

In yet further preferred embodiments, the cancer to be treated according to the present invention is endometrial cancer, preferably estrogen receptor-positive, e.g. (ER α)-positive, PR+ type I or type II ECs.

In preferred embodiments, the subject to be treated according to the present invention is a human subject. Further, the compounds for use according to the present invention can be administered orally or by injection, i.e., intramuscular injection or subcutaneous injection, wherein oral administration is preferred.

Furthermore, in specific embodiments, the compounds for use of the present invention can be administered in combination with radiation therapy and/or chemotherapy. In specific embodiment in which the compounds for use of the present invention are administered in combination with a chemotherapeutic drug, said chemotherapeutic drug is preferably selected from the group, consisting of doxorubicin, epirubicin, paclitaxel, docetaxel, cabazitaxel, mitoxantrone, estramustine, and combinations thereof, wherein docetaxel and cabazitaxel are particularly preferred.

In a related aspect, the present invention relates to a method of treating cancer in a subject, wherein the cancer is selected from the group consisting of breast cancer, prostate cancer, and endometrial cancer, comprising the step of administering to the subject an effective amount of a compound according to Formula (I): 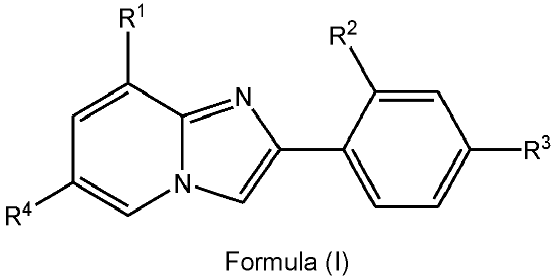 wherein
R¹ is H or Cl,
R² is H or F,
R³ is H or F, and
R⁴ is CH₃ or CFs;

In this aspect, all relevant definitions for the first aspect of the present invention equally apply. In particular, the compounds according to Formula (I), the cancer to be treated, and the specifics of administration are as defined above.

As used herein, the term "comprising"/"comprises" expressly includes the terms "consisting essentially of"/"consists essentially of" and "consisting of"/"consists of", i.e., all of said terms are interchangeable with each other herein.

Further, as used herein, the term "about" represents a modifier of ± 10% of the specified value, preferably ± 7.5%, ± 5%, ± 3%, ± 2%, or ± 1% of the specified value. Thus, by way of example, the term "about 10" includes the ranges 9 to 11, 9.25 to 10.75, 9.5 to 10.5, 9.7 to 10.3, 9.8 to 10.2, and 9.9 to 10.1.

As indicated above, the development of new antagonists of ERα and AR that do not function through the LBDs of these receptors but rather function indirectly through cofactors that enhance the activities of the receptors is needed. One example of such an approach is through molecular chaperones and cochaperones that assist the conformational folding or unfolding as well as the assembly or disassembly of other macromolecular structures. These proteins have an inherent property in recruiting coactivators that control the action of steroid receptors and present an attractive group of proteins to target.

Other studies using chemical inhibitors of the cochaperone BAG1 have been carried out and shown to decrease tumor growth. Thio-2, that docks into the BAG domain of BAG1 has been shown to inhibit proliferation of melanoma cells that have become resistant to an inhibitor of mutant BRAF. Thio-2 has also been shown to disrupt binding of BAG1L to the AR leading to inhibition of proliferation of AR positive LNCaP prostate cancer cells. However, it has recently been reported that Thio-2 inhibits prostate cancer proliferation independent of BAG1 by binding directly to the N-terminus of the AR.

An anti-tumor action of a benzothiazole-based compound A4B17 derived from Thio-2 that docks into the BAG domain of BAG1 has recently been described. This compound inhibited the proliferation of ER⁺ and AR⁺ breast and prostate cancer but not receptor negative cancer cells. It also inhibited AR target gene expression and AR⁺ prostate cancer cell growth in an *in vivo* xenograft model. However, the high concentration of this compound required for inhibition of tumor growth questions its on-target effect and warrants the development of more active inhibitors. To improve the activity of A4B17, scaffold hopping experiments were conducted focusing on heterocycle replacement to change the benzothiazole into an imidazopyridine.

Imidazopyridine scaffolds have gained significant attention in medicinal chemistry due to their frequent occurrence in a large number of marketed drug formulations and drug candidates. These compounds have shown a wide variety of biological and pharmacological activities such as anti-mycobacterial, anti-diabetic, anti-viral, and anti-cancer activities. Several *in vitro* experimental observations have shown that the anticancer effect of different imidazopyridine compounds that have been synthesized result mainly from their inhibitory effects on six main molecular mechanisms: 1) PI3K/Akt, 2) CENP-E, 3) IGF-1R, 4) CDKs, 5) tubulin polymerization, and 6) c-Met inhibition.

Herein, a group of imidazopyridine scaffold-based compounds, e.g. compound X15695 as prototype compound for this group, is described. This compound inhibited proliferation of ER⁺ and AR⁺ but not steroid receptor negative breast and prostate cancer cells. It also inhibited proliferation of an ER+, PR+ endometrial carcinoma cell line but no other tumor cells that lack ERα or AR expression. In RNA sequencing experiments in breast cancer cells, X15695 was shown to enhance the expression of aryl hydrocarbon receptor (AhR) target genes (e. g. Cyp1A1, Cyp1A2, Cyp1B1 and ALDH3A1) indicating that X15695 functions through the AhR and possibly not through ERα. Further, X15695's action on breast and prostate tumor cells was found to be through attenuation of steroid receptor signaling pathway and reactivation of wild-type and mutant p53. In breast cancer cells, X15695 exerted its action mainly through degradation of ERα expression, an action that is possibly brought about by the reported cross-talk between the AhR and ERα. X15695 also inhibited *in vivo* breast and prostate tumor xenografts when administered orally. These studies therefore identify X15695 as an oral selective estrogen and androgen receptor antagonist for the treatment of breast and prostate cancer.

Imidazopyridine derivatives known in the art target cancers and signaling molecules distinct from what is proposed in the present invention. In particular, said derivatives are structurally different from the ones of the present invention and it is therefore obvious that the signaling pathways they target are different from the ones proposed herein. The compounds of the present invention target a distinct class of cancers.

In particular, the imidazopyridine compounds of the present invention (represented by X15695) will inhibit the growth of ER⁺ and AR⁺ breast and prostate cancer cells through downregulation of ERα level and to a lesser extent AR level. Additionally, they function as orthosteric inhibitors disrupting BAG1/mortalin interaction and as a result mortalin/p53 interaction leading to nuclear translocation of p53 and enhancement of the tumor suppressor action of p53. Because it can be determined what type of tumors will respond to the present compounds, the imidazopyridine derivatives of the present invention have the potential, through the use of genetic information in clinical decision-making, to be safer and more effective drug therapies for cancer patients.

Further, the imidazopyridine compounds of the present invention (here represented by X15695) act as oral selective estrogen receptor degraders (SERDs). X15695 functions when given orally in mouse xenograft studies to degrade ERα and reactivate p53 leading to cell cycle block and the induction of apoptosis.

ER antagonists commonly used clinically are tamoxifen or fulvestrant that are classified as selective estrogen receptor modulators (SERMs) or selective estrogen receptor degraders (SERDs). Both drugs function through the ligand binding domain of ERα where several mutations occur during the course of treatment that inactivate the action of the drugs. Tamoxifen has an additional disadvantage in that it has agonistic activities in addition to its antagonistic action. Being a degrader, fulvestrant shows a superior therapeutic antagonistic activity compared to tamoxifen and has been classified as a pure antagonist. However, fulvestrant also has other weaknesses, some of which are its route of administration and bioavailability. Due to its unfavorable pharmacological properties, fulvestrant is applied via intramuscular injection. It requires two 250 mg/5ml intramuscular injections and it is slowly absorbed. Secondly, to improve fulvestrant efficacy, it is recommended to combine it with other targeted therapies such as CDK4/CDK6 inhibitors (e.g. Palbociclib) that promote cell cycle block at the G1/S phase. Combination of drugs may offer an improved action, but they are also associated with the risk of increased side effects that may limit the drugs to an extent that compromises their effectiveness.

The group of compounds of the present invention do not have the above limitations. X15695 has been shown to function effectively in xenograft experiments when given orally. X15695 also has intrinsic activity to inhibit cell cycle progression and induce apoptosis through its action on the reactivation of p53. There will therefore be no need to use it with other target therapies such as inhibitors of cell cycle progression.

To put it in perspective, there are efforts by serval pharmaceutical companies to develop oral SERDs. The present compound X15695 is a SERD that works orally in mouse tumor xenograft studies and offers an opportunity for further development for therapeutic purposes. It must be mentioned that another area of drug discovery work that is being actively pursued by several pharmaceutical companies is a search for compounds that reactivate p53. It has been shown herein that through its ability to disrupt mortalin/p53 interaction, X15695 reactivates p53, inhibits cell cycle progression and induces apoptosis. The multi-targeting of factors by one compound characterizes the uniqueness of the approach of the present invention.

The figures show:
Figure 1:
   Scaffold hopping experiment carried out with the benzothiazole A4B17 focusing on heterocycle replacement. (A) Shown are different compounds and concentrations that half maximally inhibit (IC₅₀) the clonal expansion of the prostate cell line LNCaP and the CRPC prostate cell line 22Rv.1. The potencies of the different compounds were compared with that of A4B17 (in green). X15695 (in red) was shown to be the most potent compound. (B) Chemical structures of the different classes of compounds used in the above clonogenic experiment.
Figure 2:
   Chemical structures showing the conversion of a benzothiazole scaffold into an imidazopyridine (A) and different derivatives thereof (B).
Figure 3:
   The crystal structures of X15696 (A) and X19168 (B).
Figure 4:
   Chemical structures of the benzothiazole A4B17 and six imidazopyridines that potently inhibit clonal expansion of ER⁺ and AR⁺ breast and prostate cancer cells derived from the results in Tables 1A-1B.
Figure 5:
   Comparison of the potency of the current available prostate cancer therapeutics with those of selected imidazopyridines. Quantification of the effect of the indicated concentrations of imidazopyridines on the clonal expansion of the CRPC cell line 22Rv.1. Enza and Daro stand for Enzalutamide and Darolutamide.
Figure 6:
   Volcano plots. (A)-(D) Visualization of RNA-seq analyses with Volcano plots of DEGs following X15695 treatment of MCF-7 (A, B) and T47D (C, D) cells in the absence and presence of E₂. Threshold of ILogFCI ≥ 1.0 and adj. p value ≤ 0.05 were used.
Figure 7:
   X15695 attenuates the expression of ERα and upregulates p53 expression. (A, B) Gene Set Enrichment Analysis (GSEA) plots of the top gene sets identified in a comparison of E₂ + X15695 vs E₂ (A) and X15695 vs vehicle (B) in MCF-7. (C, D) Western blot analysis of ERα in extracts of MCF-7 (C) and T47D (D) cells after treatment with vehicle or the indicated concentrations of X15695 for 48 h. Anti-β-actin antibody was used for protein loading control. (E, F) Representative immunofluorescent staining carried out on hormone starved MCF-7 cells (E) treated with 100 nM or 1 µM X15695 in the presence and absence of 10 nM E₂ for 16 h and T47D cells (F) treated with 1 µM X15695 for 16 h in full medium without hormone depletion. Staining was performed with anti-ERα antibody followed by goat anti-mouse IgG, Alexa Fluor^{™} 488 (*green*) and by DAPI *(magenta).* Fluorescence images were taken at 20×3 magnification. (G) Western blot analysis of ERα in extracts of MCF-7 cells treated with vehicle or X15695 (1 µM) for 24 h followed by treatment with 100 µg/ml cycloheximide and the termination of the reaction at the indicated time points. Anti-β-actin antibody was used to determine equal protein loading. (H) The protein signals were quantified and presented in percentages as the signal of ERα relative to the β-actin signal. The value at timepoint zero of cycloheximide treatment was set to 100%. The values are the means ± SEM, n =3. * p ≤ 0.05.
Figure 8:
   Hallmark gene set analyses showing pathways targeted by X15695. (A, B) Hallmark pathway analysis of DEGs comparing treatment with E₂ + X15695 vs E₂ and X15695 vs vehicle in MCF-7 cells or (C, D) in T47D cells.
Figure 9:

   Attenuation of ERα target gene expression by X15695. (A) Heatmaps comparing the DEGs in the estrogen signaling pathway after treating MCF-7 and T47D cells with E₂ and X15695. (B, C) Quantitative RT-PCR showing the action of X15695 on a select number of E₂ regulated genes in MCF-7 and T47D cells. Cells were serum starved for 3 days and treated with 1 µM X15695 in the presence and absence of 10 nM E₂ for 16 h. The data represents the mean ± SEM (n =4; * p ≤ 0.05; ** p ≤ 0.01; *** p ≤ 0.001; **** p ≤ 0.0001; ns is not significant).
Figure 10:
   Reactivation of p53 by X15695. (A) Heatmaps comparing the DEGs in the p53 pathway after treating MCF-7 and T47D cells with E₂ and X15695. (B, C) Quantitative RT-PCR showing the action of X15695 on a select number of p53 target genes in MCF-7 and T47D cells. Cells were serum starved for 3 days and treated with 1 µM X15695 in the presence and absence of 10 nM E₂ for 16 h. (D-I) Quantitative RT-PCR carried out to detect the expression of the indicated p53 target genes in MCF-7 cells upon X15695 treatment in the presence and absence of E₂. MCF-7 cells were serum starved for 3 days and treated with the indicated concentrations of X15695 in the presence and absence of 10 nM E₂ for 16 h. The data represents the average ± SEM (n =4; * p ≤ 0.05; ** p ≤ 0.01; *** p ≤ 0.001; **** p ≤ 0.0001; ns is not significant).
Figure 11:
   X15695 stabilizes p53. (A, B) Western blot analysis of p53 in extracts of MCF-7 (A) and T47D (B) cells treated with vehicle or the indicated concentrations of X15695 for 48 h. Anti-β-actin antibody was used for the loading control. (C, D) Quantitative RT-PCR of p53 expression in MCF-7 (C) and T47D (D) cells after treatment with vehicle (DMSO) or the indicated concentrations of X15695 for 48 h. The results are the mean ± SEM (n=3; ns represents non-significant). (E) Western blot analysis of p53 in extracts of MCF-7 cells treated with 0.5 µM MG132 in the presence and absence of 1 µM X15695 for 48 h. Anti-β-actin antibody was used for the loading control. (F) Quantification of the p53 protein band intensities relative to the intensity of the β-actin. The results are the means ± SEM (n=3; * p ≤ 0.05; ns refers to non-significant result). (G, H) Western blot analysis of Mdm2 levels in extracts of MCF7 (G) and T47D (H) cells after treatment with the indicated concentration of X15695 for 48 h. Anti-β-actin antibody was used for determination of equal protein level. The protein band intensities were quantified and presented below. (I, J) Measurement of DCF fluorescence in MCF-7 (I) and T47D (J) cells treated with the indicated concentrations of X15695 or DMSO for 48 h. The signals were normalized to DNA content of the samples measured after Hoechst staining. The results are the mean ± SEM (n=3; *** p ≤ 0.001; **** p ≤ 0.0001).
Figure 12:
   N-Acetyl cysteine abrogates ROS-mediated increase in p53 levels. X15695-mediated ROS production is impaired by prior NAC treatment. (A) Reduced 2',7'-dichlorofluorescein diacetate (H2DCF) oxidation assay showing an enhanced reactive oxygen species (ROS) production in MCF-7 cells treated with X15695 for 48 h. The results represent the averages of H2DCF oxidation normalized to DNA content (n = 1 with 6 technical replicates). (B) Western blots of lysates of MCF-7 cells treated with indicated concentrations of N-Acetyl cysteine (NAC) 1 h prior to treatment with 1 µM X15695 or DMSO for 48 h. Expression of p53 was monitored using anti-p53 antibody and anti-β-actin antibody was used as a protein loading control.
Figure 13:
   Cellular localization of p53 in the absence and presence of X15695. (A, B) Immunofluorescence images showing p53 localization in MCF-7 and T47D cells treated with vehicle (DMSO) and 1 µM X15695 for the indicated time periods. For staining, anti-p53 antibody was used followed by goat anti-mouse IgG, Alexa Fluor^{™} 488 antibody - *green*) and DAPI (*magenta*)*.*
Figure 14:

   X15695 reactivates p53. (A, B) Representative immunofluorescence microscopy images showing cellular localization of p53 and mortalin in MCF-7 (A) and T47D (B) cells treated with 1 µM X15695 or vehicle (DMSO) for 16 h. Staining was performed using anti-p53 (followed by incubation with goat anti-rabbit IgG, Alexa Fluor^{™} 546 antibody - red), anti-mortalin (followed by goat anti-mouse IgG, Alexa Fluor^{™} 488 antibody incubation - *green)* and DAPI (*light blue*). (C) Western blot analysis of mortalin and p53 in immunoprecipitated extracts of MCF-7 cells treated with 1 µM X15695 for 48 h. 50 µg of cell extract was used as input control while 1,000 µg was immunoprecipitated with anti-p53 or IgG antibodies immobilized on protein A/G agarose beads. (D) Western blot analysis of mortalin and BAG1 in immunoprecipitated extracts of MCF-7 cells treated with 1 µM X15695 for 48 h. MCF-7 cells were treated and processed as in (C) except that the immunoprecipitation was carried out with a mouse anti-BAG1 antibody. (E, F) Cell cycle analysis of MCF-7 (E) and T47D (F) cells treated with vehicle or 1 µM X15695 for 48 h. 1×10⁶ cells were stained with 7-AAD and used for flow cytometric measurement. Data was analyzed by FlowJo software. (G, H) Apoptosis assessment of MCF-7 (G) and T47D (H) cells treated with 1 µM X15695 for 48 h. The cells were double stained with 7-AAD and Annexin V. Cells stained with 7-AAD or Annexin V alone were used for compensation of double staining. 1×10⁶ cells were used for the flow cytometric assay.
Figure 15:
   X15695 disrupts BAG2 complex with p53^{L194F}. Western blot analysis of BAG2 and BAG5 in immunoprecipitated extracts of T47D cells treated with 1 µM X15695 for 48 h. 70 µg of cell extracts was used as input control while 1,000 µg was immunoprecipitated with anti-p53 or IgG antibodies immobilized on protein A/G agarose beads.
Figure 16:
   Knockdown of p53 attenuates X15695-mediated decrease in cell viability. Cells transfected with control or p53 siRNA for 16 h were treated with vehicle or X15695 for 24 h or 72 h for Western blot or MTT assay. (A) Western blot analysis of p53 protein after transfecting MCF-7 and T47D cells with control and p53 siRNA and treatment with vehicle or X15695 (1 µM) for 24 h. β-actin was used for the loading control. (B) Cell viability determination using MTT assay in control and p53 siRNA transfected cells treated with vehicle or X15695 (1 µM) for 72 h (n ≥3; *p ≤ 0.05).
Figure 17:
   X15695- and fulvestrant-mediated inhibition of ERα expression. (A) Western blot analysis of ERα in the extracts of MCF-7 cells treated with 1 µM of fulvestrant or X15695 or DMSO (Vehicle) at indicated time points. β-actin was used for the loading control. The protein signals were quantified and presented in percentages as the signal of ERα relative to the β-actin signal (*lower panel*)*.* The value at zero time of treatment was set to 100%. The values are the mean ± SEM (n =3. * p ≤ 0.05, ** p ≤ 0.01; *** p ≤ 0.001). (B) Western blot analysis of ERα in the extracts of MCF-7 cells treated with increasing concentration of fulvestrant or X15695 for 8 h. β-actin was used for the loading control. The protein signals were quantified and presented in percentages relative the vehicle-treated sample (*lower panel*)*.* The values are the mean ± SEM (n =3; **** p ≤ 0.0001).
Figure 18:
   X15695 decreases the viability of tamoxifen-resistant MCF-7 cells and inhibits tumor formation in a mouse tumor xenograft model. (A) Measurement of cell viability by MTT assay of tamoxifen resistant cells (TRMCF-7) treated with the indicated concentrations of X15695, fulvestrant and tamoxifen for 48 h. Five replicates were used for each experiment, and the experiment was repeated 3 times (** p ≤ 0.01, *** p ≤ 0.001, **** p ≤ 0.0001, ns = non-significant). (B) Representative image of a clonogenic assay showing the effect of the indicated concentrations of X15695, fulvestrant and tamoxifen on clonal expansion of tamoxifen-resistant MCF-7 cells. (C) Quantification of colonies in the clonogenic assay described in (B). (D) Representative images of some of the tumors obtained in a mouse MCF-7 tumor xenograft after treatment with vehicle and X15695. (E) Tumor volumes of established subcutaneous MCF-7 xenografts treated orally daily with vehicle or X15695 (30 mg/kg body weight). The values are the mean ± SEM (* p= 0.05; n =14). (F) Tumor weights at the end of treatment were recorded and presented in the bar chart. The values are the means ± SEM (* p= 0.05; n =14). (G) Mouse body weights measured twice weekly for all animals over 16 days. Presented are the mouse body weight changes for each group in percentages. (H) Western blots analysis of ERα and p53 from the extracts of select tumors at the end of treatment. Anti-vinculin antibody was used to determine the level of protein loaded. (I, J) Quantification of ERα (I) and p53 (J) protein levels in select tumors relative to the vinculin loading control. The results are the mean ± SEM. * p ≤ 0.05; *** p ≤ 0.001; n = 12.
Figure 19:
   X15695-mediated regulation of AR action in prostate cancer cells. (A)-(C), GSEA plots of the topmost signaling pathways and heatmaps of Log2 fold-change in gene expression in the comparison of DHT + X15695 vs DHT and X15695 vs vehicle in LNCaP cells.
Figure 20:

   X15695 and its derivatives attenuate AR target gene expression. Quantitative RT-PCR was carried out with primers of the indicated genes in LNCaP cells serum-starved for 3 days and treated with 10 nM DHT and X15695 (5 µM) and the indicated imidazopyridine compounds for 16 h. RNA was extracted from the treated cells and RT-PCR was carried out. The results are the means ± SEM (n > 4; * p ≤ 0.05; ** p ≤ 0.01; *** p ≤ 0.001; **** p ≤ 0.0001; ns is not significant).
Figure 21:
   X15695 functions synergistically with chemotherapeutics drugs in the inhibition of proliferation of prostate cancer cell lines. (A, B) 22Rv.1 and LNCaP cancer cells or (C) LNCaP cells were treated with the indicated concentration of the chemotherapeutic drugs cabazitaxel or docetaxel in the absence and presence of the indicated concentration of X15695. Shown are the effect of the different compounds on the clonal expansion of the cells.
Figure 22:
   X15695-mediated regulation of expression and cellular localization of p53 in prostate cancer cells. (A) Western blot analyses of p53 levels in extracts of the prostate cancer cells 22Rv.1, LNCaP and LAPC-4 treated with 1 µM of the indicated imidazopyridines for 48 h. The blots were carried out with cell lysate using anti-p53 and anti-β-actin antibodies. (B) Immunofluorescence images showing the cellular localization of p53 after treating 22Rv.1, LAPC-4 and LNCaP cells with 1 µM X15695 or vehicle (DMSO) for the indicated time period. Staining was performed with anti-p53 antibody followed by incubation with goat anti-mouse IgG, Alexa Fluor^{™} 488 antibody (red) and DAPI (*blue*)*.* (C) 22Rv.1, LAPC-4 and LNCaP cells were transfected with control and p53 siRNA for 16 h and treated with vehicle or X15695 (5 µM) for 24 h. Western blot analysis was performed with anti-p53 and anti-β-actin antibodies. (D) MTT cell viability assay carried out with 22Rv.1, LAPC-4 and LNCaP cells previously transfected with control and p53 siRNA and treated with vehicle and X15695 for 72 h. The results are the mean ± SEM (n =3; *p ≤ 0.05).
Figure 23:
   X15695 induces cell cycle arrest but no apoptosis in the inhibition of prostate cancer cell growth. (A, B) Cell cycle profile measured by flow cytometric assay of LNCaP (A) and LAPC-4 cells (B) treated with vehicle or 1 µM X15695 for 48 h. Cells for flow cytometry were stained with DRAQ5. (C, D) LNCaP and (E, F) LAPC4 cells were incubated in medium alone (-) or with the indicated concentrations of X15695, DMSO or Etoposide (ETP; 250 µM) for 48 h. Subsequently, cells were stained with Hoechst and propidium iodide (PI). Images were obtained by automated microscopy and analyzed by the scan^R software. (C, E) Total cell number of LNCaP and LAPC4 cells divided into living and dead cells after treatment with indicated concentrations. (B, D) Different types of dead cells presented as percentage of the total cell number. The error bars are SEM values related to the total cell number (A, C) or the percentage of dead cells (D, F).
Figure 24:
   X15695 inhibits prostate cancer tumor growth. (A) Representative examples of LAPC-4 xenograft mouse tumors after daily treatment with vehicle, X15695 (10 and 30 mg/kg) and enzalutamide (10 mg/kg) for 43 days. (B) Tumor weights recorded at the end of treatment and presented in the bar chart. The values are the means ± SEM expressed (**p ≤ 0.01; **** p ≤ 0.0001; n =14; ns is not significant). (C) Mouse body weights measured twice weekly over the 43 treatment days. Presented are the mouse body weight changes, in percentages, for each group.
Figure 25:
   X15695 is a selective inhibitor of clonal expression of ER⁺ breast cancer cells and endometrial carcinoma cells. Quantification of the effect of the indicated concentrations of X15695 on clonal expansion of the different tumor cells. n.d. is non-determinable. The different cancer cell lines are A549: alveolar adenocarcinoma, U2OS: osteosarcoma, MCF-7: breast cancer, HeLa: cervical cancer, Ishikawa: endometrial cancer, HepG2: hepatoma, Panel: pancreatic cancer, MxCP3: pancreatic cancer, HCT116: colorectal carcinoma.

The present invention will be further illustrated by the following examples without being limited thereto.

### Examples

### Material and methods:

### Cell lines

All cell lines were obtained from the American Type Culture Collection and their identities were confirmed by short tandem repeat profiling (BioSynthesis, Lewisville, TX and DSMZ Braunschweig, Germany). All cell lines were confirmed to be mycoplasma negative, using the Venor^{®}GeM Classic Mycoplasma Detection Kit for conventional PCR (Minerva Biolabs, Berlin, Germany). MCF-7, T47D, ZR-75-1, MDA-MB231, Ishikawa, LNCaP, 22Rv.1, PC3, DU145 and LAPC-4 cells were cultured in RPMI1640 supplemented with 10% FBS, 1% penicillin/streptomycin at 37°C in an incubator with 5% CO₂ and 90% humidity. LAPC-4 cells were additionally supplemented with 1 nM dihydrotestosterone (DHT). HCT116 was cultured in McCoy's 5A medium and Panc1 and BxPC3 were cultured in Dulbecco's Eagles modified medium supplemented with 10% FBS, 1% penicillin/streptomycin. For experiments requiring hormone starvation, cells were cultured for72 h in phenol red-free RPMI 1640 medium, supplemented with 3% charcoal-stripped fetal calf serum (CCS).

### p53 siRNA knockdown and Western blotting

Transfection experiments with siRNA were performed using Lipofectamine 2000 (Life Technologies, Thermo Fisher Scientific, Karlsruhe, Germany) according to the manufacturer's instructions using SMARTpool ON-TARGETplus siRNA against p53 or human BAG1 from Dharmacon (Cambridge, UK). Briefly, 5×10⁴ cells were transfected with 6 µl of Lipofectamine 2000 and 10 nM p53 siRNA or control siRNA (5'-GGUGCGCUCCUGGACGUAGCC-3', SEQ ID NO: 1). Sixteen hours after transfection, the medium was changed and X15695 or vehicle was added to the cells for 72 h for proliferation assay or 24 h for protein analysis.

For Western blotting, cells were lysed in NP-40 lysis buffer (1% NP-40, 50 mM Tris-HCl, pH 8.0, 150 mM NaCl, 5 mM EDTA) or 1× passive lysis buffer (Promega, Germany) for 15 min on ice. Cell debris was removed by centrifugation at 13,000 rpm for 10 min and used for SDS-polyacrylamide gel electrophoresis (PAGE) and Western blotting.

Western Blotting was carried out using standard protocols with antibodies: p53 (clone DO1), p53 (Sp5, Thermo Fisher Scientific Karlsruhe, Germany), Mdm2 (4B2, Abcam, Cambridge, UK). BAG1 (CC9E8), β-actin (C4), vinculin (H-10), GAPDH (G9), Tubulin (B7), ERα (F-10), Mortalin (D-9), AR (441), BAG2 (C-6), BAG5 (F-9) and BAG3 (19) were all purchased from Santa Cruz (Heidelberg, Germany). Secondary goat anti-mouse and goat anti-rabbit antibodies were purchased from DAKO (Bollschweil, Germany).

### Cycloheximide chase and MG132 stability assay

Unless otherwise stated, cycloheximide chase and MG132 stability experiments were performed as follows: 2×10⁵ MCF-7 cells were seeded in 6 cm dishes and treated with 1 µM X15695 or the equivalent volume of DMSO for 24 h. Thereafter, cells were incubated with the protein synthesis inhibitor cycloheximide (100 µg/ml) and harvested at time points 0, 30, 60, 90, and 120 min. Cells were lysed and used for Western blotting to determine the ERα level at each time point. For MG132 experiments, MCF-7 cells were similarly treated but in the presence and absence of the proteasome inhibitor MG132 (0.5 µM) for 48 h. Cells were harvested and lysed for Western blot assay to detect the p53 level at each condition.

### Co-immunoprecipitation

For co-immunoprecipitation experiments, 1×10⁶ cells were seeded in 15 cm dishes and treated with 1 µM X15695 or the equivalent volume of DMSO for 48 h. Cells were harvested, washed with PBS, and lysed using NP-40 lysis buffer. Protein A agarose beads (Thermo Fisher Scientific) were mixed with protein G agarose beads (Thermo Fisher Scientific) (4:1) and preincubated overnight at 4°C with anti-p53 or anti-BAG1 or control IgG antibody. Cellular extracts from X15695 treated and untreated cells (1,000 µg) were incubated with antibody-preincubated protein A/G agarose beads for 3 to 4 h at room temperature. The beads with immunoprecipitated proteins were collected at 4°C by centrifugation at 1,000 g for 5 min and washed 4 to 5 times with NP-40 lysis buffer. The immunoprecipitated proteins were eluted by heating in SDS sample buffer for 5 min at 95°C and resolved on SDS-PAGE. Thereafter, they were subjected to Western blot analysis to detect mortalin, BAG1, BAG2, BAG5 and p53. Cellular extracts containing 50 to 70 µg protein from X15695 treated and untreated cells were used as the input control for the targeted proteins.

### RNA sequencing and quantitative RT-PCR experiments

MCF-7, T47D and LNCaP cells (5×10⁴ each) were seeded in 6-well plates and cultured with hormone-depleted medium for 3 days. Subsequently, the MCF-7 and T47D cells were treated with 10 nM 17-β-estradiol (E₂) or dihydrotestosterone (DHT) for the LNCaP cells, 1 µM X15695 (for the MCF-7 and T47D cells) or 5 µM for LNCaP cells and X15695 together with E₂ or DHT. A 1 h pre-treatment with X15695 was performed prior to treatment with E₂ or DHT for 16 h. RNA was extracted using innuPREP RNA mini Kit 2.0 (Analytic Jena, Berlin, Germany). 1 µg RNA per sample was used as input material for the RNA sample preparations. Sequencing libraries were generated using NEBNext^{®} UltraTM RNA Library Prep Kit for Illumina^{®} (NEB, USA) following the manufacturer's recommendations and index codes were added to attribute sequences to each sample. The clustering of the index-coded samples was performed on a cBot Cluster Generation System using PE Cluster Kit cBot-HS (Illumina) according to the manufacturer's instructions. After cluster generation, the library preparations were sequenced on an Illumina platform and paired-end reads were generated. Library preparation and sequencing were carried out by Novogene Europe, Cambridge, UK. Differential expression analysis was performed using R package Limma (v3.52.2) and visualized with R package gplots (v 3.1.3) with Log2 fold changes ≥1 and p(FDR)≤0.05). Gene Ontology (GO) analysis and Gene set enrichment analysis was performed using R package GSEABase (v1.58.0) and GSVA (v1.44.2) and the results were visualized by R package enrichplot (v1.16.1). The data was deposited at the GEO repository under accession number GSE218556. Select target genes were analyzed by RT-PCR experiments with RNA from the samples used for the RNA-seq analysis. PCR analysis was carried out using the SYBR Green GoTaq PCR mix (Promega, Walldorf, Germany) and Rib36B4 as a housekeeping gene. The sequence of the primers used are as follows:

| | |
|---|---|
| Rib36B4 | For 5'-CTCCTGAGCGCAAGTACTCC-3' (SEQ ID NO: 2) |
| | Rev 5'-GTCACCTTCACCGTTGTTCCA-3' (SEQ ID NO: 3) |
| KLK3 | For 5'-CCCGGTTGTCTTCCTCACCC-3' (SEQ ID NO: 4) |
| | Rev- 5'-GCCTCCCACAATCCGAGACA-3' (SEQ ID NO: 5) |
| F5 | For 5'-TCCAGGCCGAGAATACACCTA-3' (SEQ ID NO: 6) |
| | Rev 5'-CGATTTGCTTGTCAAACGTCTTC-3' (SEQ ID NO: 7) |
| DUOX1 | For 5'-GTGCTCCCTCTGTTGTTCGT-3' (SEQ ID NO: 8) |
| | Rev 5'-GCTTCTCAGACACGATGCTCT-3' (SEQ ID NO: 9) |
| MICAL1 | For 5'-ATGGGCAGCCTGATGTCTCT-3' (SEQ ID NO: 10) |
| | Rev 5'-GGCGCCATGCTTCTCTTG-3' (SEQ ID NO: 11) |
| SAT1 | For 5'-GCCGACTGGTGTTTATCCGT-3' (SEQ ID NO: 12) |
| | Rev 5'-TTTCTTCCCTTTGCGGACCA-3' (SEQ ID NO: 13) |
| FKBP5 | For 5'-TTCAAGGGAGGCAAATACATG-3' (SEQ ID NO: 14) |
| | Rev 5'-TCCAAGGGCCTTGTCACAG-3' (SEQ ID NO: 15) |
| SLC7A11 | For 5'-ATGCAGTGGCAGTGACCTTT-3' (SEQ ID NO: 16) |
| | Rev 5'-CATGGAGCCAAAGCAGGAGA-3' (SEQ ID NO: 17) |
| OSGIN | For 5'-AGAAGAAGCGAAGAGGTC-3' (SEQ ID NO: 18) |
| | Rev 5'-CGGACACAAAGTTATGCC-3' (SEQ ID NO: 19) |
| p53 | For 5'-TGCGTGTTTGTGCCTGTCCT-3' (SEQ ID NO: 20) |
| | Rev 5'-GTGCTCGCTTAGTGCTCCCT-3' (SEQ ID NO: 21) |
| CDKN1A(p21) | For 5'-GACTCTCAGGGTCGAAAACG-3' (SEQ ID NO: 22) |
| | Rev 5'-GCGGATTAGGGCTTCCTCTT-3' (SEQ ID NO: 23) |
| GADD45A | For 5'-CTCAACGTCGACCCCGATAA-3' (SEQ ID NO: 24) |
| | Rev 5'-GCCTGGATCAGGGTGAAGTG-3' (SEQ ID NO: 25) |
| BB3 (PUMA) | For 5'-GACCTCAACGCACAGTACGAG-3' (SEQ ID NO: 26) |
| | Rev 5'-AGGAGTCCCATGATGAGATTGT-3' (SEQ ID NO: 27) |
| FAS | For 5'-ACACCAAGTGCAAAGAGGAAGGAT-3' (SEQ ID NO: 28) |
| | Rev 5'-GACCAAGCTTTGGATTTCATTTCTG-3' (SEQ ID NO: 29) |
| PGR | For 5'-CTTAATCAACTAGGCGAGAG-3' (SEQ ID NO: 30) |
| | Rev 5'-AAGCTCATCCAAGAATACTG-3' (SEQ ID NO: 31) |
| TFF1 | For 5'-CAATGGCCACCATGGAGAAC-3' (SEQ ID NO: 32) |
| | Rev 5'-AACGGTGTCGTCGAAACAGC-3' (SEQ ID NO: 33) |
| GREB1 | For 5'-GAGTGACAATGAGGAAGAG-3' (SEQ ID NO: 34) |
| | Rev 5'-CTCGTTGGAAATGGAGACAA-3' (SEQ ID NO: 35) |
| AREG | For 5'-TGAGATGTCTTCAGGGAGTG-3' (SEQ ID NO: 36) |
| | Rev 5'-AGCCAGGTATTTGTGGTTCG-3' (SEQ ID NO: 37) |
| AGR2 | For 5'-GACCAAGCTTTGGATTTCATTTCTG-3' (SEQ ID NO: 38) |
| | Rev 5'-CAGGTTCGTAAGCATAGAGACG-3' (SEQ ID NO: 39) |
| PDZK1 | For 5'-GCAGGCTCAGAACAGAAAGG-3' (SEQ ID NO: 40) |
| | Rev 5'-TCCAGGGTTTCCACAGACTC-3' (SEQ ID NO: 41) |

### MTT (3-(4,5-dimethylthiazol-2-yl)-2,5-diphenyltetrazolium bromide) cell viability assay

Cells were seeded in duplicates at a density of 1×10³ cells/well in a 24-well plate format and incubated for 6 days in culture medium. Cells transfected with siRNA were incubated for 72 h after X15695 addition. Thereafter, the medium was exchanged for a fresh medium containing 0.5 mg/ml MTT (Sigma-Aldrich) and incubated at 37°C for 1 h to 4 h (depending on cell line) until intracellular purple formazan crystals were formed. The MTT solution was removed, and the purple crystals were dissolved in isopropanol and the optical absorbance at 595 nm was recorded using SpectraMax iD3, Molecular Devices, San Jose, CA, USA.

### Clonogenic assay

Cells were seeded at a density of 1-2×10³ cells/well in a 6 well plate in RPMI 1640 supplemented with 10% FBS, penicillin (100 u/ml), streptomycin (100 u/ml), and L-glutamine (2 mM). Cells were treated with increasing concentrations of X15695 or the indicated compound and cultured for 14 to 21 days. Cabazitaxel and docetaxel were purchased form Sigma-Aldrich. Medium and compounds were exchanged after 7 days. Cells were fixed with methanol/ acetic acid mixture (3:1 v/v) and the formation of colonies was visualized using 0.5% crystal violet (w/v) in 20% methanol (v/v). Plates were scanned in a conventional office scanner (Epson). The area covered by colonies was calculated using the ColonyArea Plugin for Imaged.

### Reduced 2',7'-dichlorofluorescein (DCF) diacetate oxidation assay

For measurements of ROS levels, MCF-7 and T47D cells were seeded at 1×10³ cells in RPMI 1640 supplemented with 10% FBS and treated with 10⁻⁹ - 10⁻⁶ M X15695 for 48 h. Afterwards, cells were loaded with 10 µM H2DCF-DA (Thermo Fisher Scientific, Karlsruhe, Germany) in phenol red-free medium for 40 min. Cells were subsequently washed with medium and further incubated in medium for 120 min before analysis of fluorescence at 485 nm excitation and 530 nm emission using a multi-well fluorescence reader (SpectraMax iD3, Molecular Devices with SoftMax Pro 7 software, Biberach an der Riss, Germany). After the DCF assay, cells were frozen at -80°C overnight. After thawing, DNA was stained with a Hoechst dye (10 µg/ml final concentration) for 30 min at room temperature and fluorescence was measured at 352 nm excitation and 454 nm emission in a multi-well fluorescence reader (SpectraMax iD3, Molecular Devices with SoftMax Pro 7 software, Biberach an der Riss, Germany). DCF fluorescent intensities were normalized to Hoechst fluorescent intensities.

### Immunofluorescence assay

For immunofluorescence assay, MCF-7, T47D, 22Rv.1, LAPC-4 and LNCaP cells (10⁴ each) were seeded on coverslips in a 24-well plate and the desired treatment applied. After three times washing with PBS, cells were fixed with 4% paraformaldehyde solution for 10 min at room temperature. Next, the cells were washed three times with PBS and permeabilized with 0.5% Triton X-100 for 10 min at room temperature. Following three additional washing steps with PBS, cells were treated with 5% bovine serum albumin (w/v) for 1 h at room temperature and subjected to overnight incubation with the primary antibody p53 (DO1 clone or Sp5, Thermo Fisher Scientific), ERα (F-10, Santa-Cruz) or mortalin (D-9, Santa-Cruz) and then washed three times with PBS followed by incubation with goat anti-mouse IgG, Alexa Fluor^{™} 488 antibody and/or goat anti-rabbit IgG, Alexa Fluor^{™} 546 antibody (Thermo Fisher Scientific) for 1 h at room temperature in the dark. All subsequent steps were performed in the dark. Cells were washed 3 times with PBS and stained with 0.1 µg/ml DAPI (Thermo Fisher Scientific) solution at 1:10,000 (v/v) dilution for 15 min at room temperature. After three times washing with PBS the cells were mounted in Mowiol^{®} 4-88 on microscope slides and observed by laser-scanning confocal microscopy (Confocal Microscope platform STELLARIS6-LSM900, Leica, Germany).

### Flow cytometry assay

LNCaP, LAPC-4, MCF-7 and T47D cells were treated with 1 µM X15695 or DMSO for 48 h. Single cell suspensions (1×10⁶ cells) were prepared in 150 µl PBS/10% FBS. Cells were fixed by addition of 5 ml ice-cold ethanol (70%) and over-night incubation at 4°C. Fixed LNCaP and LAPC-4 cells were stained with 20 µM DRAQ5 (5mM stock, Thermo Fisher Scientific) and incubated for 15 min at room temperature. Fixed MCF-7 and T47D cells were resuspended in PBS at concentration of 1×10⁶ cells/100 µl and stained by addition of 5 µl of 7-AAD (BD Bioscience, San Diego, CA, USA) for 15 min at room temperature in the dark. Flow cytometry analysis was performed using BD FACSAria (BD Biosciences, Franklin Lakes, NJ, USA). Doublets and dead cells were excluded from the analysis. Flow cytometry data were analyzed using FlowJo 10.8.1 cytometry analysis software (FlowJo, LLC).

Induction of apoptosis in response to treatment with X15695 was analyzed in MCF-7 and T47D cells using PE Annexin V Apoptosis Detection Kit with 7-AAD (BD Bioscience, San Diego, CA, USA) according to the manufacturer's protocol. In short, cells were harvested by trypsinization, and single cell suspensions (1×10⁶ cells) were prepared as described above in 100 µl PBS. Cells were stained with 5 µl PE Annexin V and 5 µl 7-AAD for 15 min at room temperature in the dark. Subsequently, 400 µl PBS were added to each sample and flow cytometry measurements were performed within one hour. Cells stained with PE Annexin V or 7-AAD alone were used for compensation. Flow cytometry analysis was performed using BD FACSAria (BD Biosciences, Franklin Lakes, NJ, USA). Flow cytometry data were analyzed using FlowJo 10.8.1 cytometry analysis software (FlowJo, LLC).

Apoptosis in LNCaP and LAPC-4 cells was measured using an automated high-throughput microscopy method known in the art. In brief, LNCaP and LAPC4 cells (5×10³) were seeded in a 96-well plate format. Cells were treated with 10⁻⁹ - 10⁻⁶ M X15695, DMSO or etoposide (250 µM) for 48h. Cells were stained with Hoechst 33342 (0,15 µg/ml) and propidium iodide (PI) (0,25 µg/ml) for 30 min at 37°C. Four brightfield and fluorescence images were acquired from different positions in each well using an automated Olympus IX81 fluorescence microscope (Olympus, Hamburg, Germany). The Hoechst dye was detected at excitation 350 nm and emission 450 nm and PI dye was detected at 488 nm and 590 nm. The total number of cells (all Hoechst-stained nuclei) as well as the number of early apoptotic, late apoptotic, and necrotic cells (combination of Hoechst and PI nuclei intensity) was obtained by analyzing images with the scan^R software (version 2.7.3, Olympus, Hamburg, Germany).

### Mouse xenograft experiments

All animal experiments were performed according to European and German statutory regulations and approved by the Regierungspräsidium Karlsruhe, Germany.

MCF-7 and LAPC-4 cells (2×10⁶) were suspended in 100 µl PBS: Matrigel (Corning NY USA, 1:1) and injected subcutaneously into both flanks of 6-7 weeks old female (for MCF-7 cells) and male (for LAPC-4 cells) athymic nude-Foxn1^{nu} mice (Envigo RMS GmbH, Düsseldorf, Germany). When tumor volumes were around 50 to100 mm³, mice were randomized into four groups (7 mice per group) and treated daily by oral gavage as follows: (1) Control group (Vehicle) (corn oil, 1.0% dimethyl sulfoxide (DMSO) and (2) X15695 group, 30 mg per kg body weight) for the MCF-7 tumors or (1) Control group (Vehicle) (corn oil, 1.0% dimethyl sulfoxide (DMSO) (2) enzalutamide (Enza group, 15 mg per kg body weight), (3) X15695 group, 30 mg per kg body weight) and, (4) X15695 group (15 mg per kg body weight) for the LAPC-4 tumors. Body weight and tumor volumes were measured twice a week. Tumor volume was measured with a digital vernier caliper and calculated using the formula vol = length × width × height × 0.5236 and recorded or calculated relative to the starting volume which was nominally set at 100%. After 16 days for the MCF-7 tumors or 42 days for the LAPC-4 tumors, mice were sacrificed and the tumors removed, photographed, and weighed. Percent body weight change was calculated using the following formula: group percent weight change = [(new weight - initial weight)/initial weight] × 100]. Tumors were snap-frozen and stored for Western blot analysis.

### Synthesis of imidazopyridine derivatives - General remarks

The starting materials and reagents were purchased from abcr, Acros, Alfa Aesar, ChemPUR, Fluka, Fluorochem, Merck, Sigma Aldrich, Strem, TCI, or Thermo Fisher Scientific and used without further purification unless stated otherwise. Solvents of technical grade were purified via distillation prior to use (ethyl acetate, dichloromethane, cyclohexane), solvents of *p.a.* quality were purchased from Acros, Fisher Scientific, Sigma Aldrich, Roth, or Riedel-de Haën and were used without further purification. Air- and moisture-sensitive reactions were carried out under nitrogen or argon atmosphere in oven-dried glassware using standard Schlenk techniques.

Reactions in vials were sealed with Crimp caps; both vials and caps were purchased at Chroma Globe. Solvents were evaporated under reduced pressure at 40°C using a rotary evaporator. For solvent mixtures, each solvent was measured volumetrically.

### Synthesis of imidazopyridine derivatives - Flash chromatography

Purifications via flash chromatography were performed using silica gel (SiO₂, 0.040 mm × 0.063 mm, Merck) and quartz sand (glowed and purified with hydrochloric acid). After removing the solvent under reduced pressure, the crude products were immobilized on Celite (Sigma Aldrich) and applied to the column as a solid.

For automatic flash chromatography, an Interchim PuriFLASH XS 400, Interchim PuriFLASH 4125 or Interchim PuriFLASH 5.125 was used in combination with hand-packed silica columns (SiO₂, 0.040 mm × 0.063 mm, Merck) as well as prepacked SIHP (silica high performance, 15 µm, 4 g/12 g/40 g/80 g) columns from Interchim. Fractions were separated and collected using a diode array detector (DAD).

### Synthesis of imidazopyridine derivatives - Thin-layer chromatography (TLC)

Reactions were monitored by thin-layer chromatography (TLC) using silica-coated aluminum plates (Merck, silica gel 60, F₂₅₄). UV active compounds were detected with a UV-lamp (Hanau Quarzlampen, Typ 204 AC) at 254 nm and 366 nm excitation. Moreover, Seebach solution (2.5 % phosphomolybdic acid, 1.0% Cerium(IV)sulphate tetrahydrate, 6.0% conc. H₂SO₄ in H₂O) with subsequent heating of the TLC plate was used to stain the spots. Liquid-Chromatography Mass Spectrometry (LC-MS) was conducted using a device from Agilent with HP 1100 MSD G1946 Mass Detector and a Kinetex XB-C18 column (2.6 µm, 100 × 4.60 mm) from Phenomenex. API-ES was used as a method of ionization and the following program was applied:
*10_99_P* (positive polarity): injector volume 10.0 µl, flow rate 1.0 ml/min, run time 20.0 min, solvent: water (bidistilled) 50%, acetonitrile 20%.

### Synthesis of imidazopyridine derivatives - Melting points

Melting points were detected on an OptiMelt MPA100 device from Stanford Research System.

### Synthesis of imidazopyridine derivatives - Nuclear Magnetic Resonance Spectroscopy (NMR)

A Bruker Ascend 400 was used to record NMR spectra; ¹H-NMR spectra were measured at 400 MHz, ¹³C-NMR spectra at 100 MHz and ¹⁹F-NMR spectra at 376 MHz. All measurements were conducted at room temperature using CDCl₃ or DMSO-*d*₆ acquired from Eurisotop and Sigma Aldrich as solvents and accordingly referenced to CDCl₃ (¹H 7.27 ppm, s / ¹³C 77.0 ppm, t) or DMSO-*d*₆ (¹H 2.50 ppm, s / ¹³C 39.52 ppm, sep).

Chemical shifts are given in ppm (parts per million) and the spectra were analyzed following first order spectra. The signal area was given for multiplets whereas the signal center was used for centrosymmetric signals. The signal splitting was characterized using the following abbreviations: s (singlet), d (doublet), t (triplet), q (quartet), m (multiplet), bs (broad singlet), dd (doublet of the doublet), td (triplet of the doublet) etc. For ¹³C spectra the peaks were observed as singlet if not specifically stated otherwise.

Coupling constants *J* are given in Hz (Hertz) and the number of bonds between the coupling cores is indicated as superscripted index in front of the coupling constant. Signals of the ¹³C spectra were assigned using DEPT90 and DEPT135 spectra (distortionless enhanced polarization transfer) as well as HSQC (heteronuclear single quantum coherence) and HMBC (heteronuclear multiple bond correlation).

### Synthesis of imidazopyridine derivatives - Infrared Spectroscopy (IR)

IR spectra were measured via ATR (Attenuated Total Reflection) on a Bruker IFS 88. The positions of the absorption bands are given in wavenumbers *ṽ* in cm⁻¹ and were measured in the range from 3600 cm⁻¹ to 500 cm⁻¹.

Characterization of the absorption bands was done in dependence of the absorption strength with the following abbreviations: vs (very strong, 0-9%), s (strong, 10-39%), m (medium, 40-69%), w (weak, 70-89%), vw (very weak, 90-100%).

### Synthesis of imidazopyridine derivatives - Mass Spectrometry (MS)

EI-MS (electron ionization mass spectroscopy) and FAB-MS (fast atom bombardment mass spectrometry) were conducted on a Finnigan Mat 95 with 3-nitrobenzyl alcohol (3-NBA) as matrix and reference for high resolution. The intensity of the signals is given relative to the intensity of the highest peak (100%). For the interpretation of the spectra, molecular peaks [M]+, peaks of protonated molecules [M+H]⁺ and characteristic fragment peaks are indicated with their mass-to-charge ratio (m/z) and their intensity in percent, relative to the base peak (100%) is given. In case of high-resolution measurements, the maximum tolerated error is ±5 ppm.

ESI-MS (electron spray ionization mass spectrometry) was conducted with a ThermoFisher Q Exactive Plus in positive mode with a voltage of 4kV. The tolerated error is ±5 ppm of the molecular mass. The spectra were interpreted by molecular peaks [M]⁺, peaks of protonated molecules [M+H]⁺ and characteristic fragment peaks and indicated with their mass-to-charge ratio (*m*/*z*).

### Synthesis of imidazopyridine derivatives - Elemental Analysis (EA)

Elemental analysis was conducted using an Elementar vario Micro and a Sartorius M2P analytical balance. Calculated and found percentage for carbon (C), hydrogen (H), sulfur (S) and nitrogen (N) are indicated in fractions of 100%.

### Synthesis of imidazopyridine derivatives - High Performance Liquid Chromatography (HPLC)

Preparative Reversed Phase High Performance Liquid Chromatography (RP-HPLC) was performed on the PuriFLASH 4125 system from Interchim. A VDSpher^{®} C18-M-SE precolumn (10 µm, 40 × 16 mm) followed by a PuriFLASH C18-AQ separation column (10 µm, 250 × 21.2 mm) was used as the stationary phase. A gradient of acetonitrile and double distilled water at a flow rate of 15 mL/min served as the mobile phase.

### Synthesis of imidazopyridine derivatives - Single Crystal X-Ray Diffraction (XRD)

Crystallographic data for compounds X15696 and X19168 reported herein have been deposited with the Cambridge Crystallographic Data Centre as supplementary information no. CCDC-2218538 and 2218539. Copies of the data can be obtained from https://www.ccdc.cam.ac.uk/structures/.

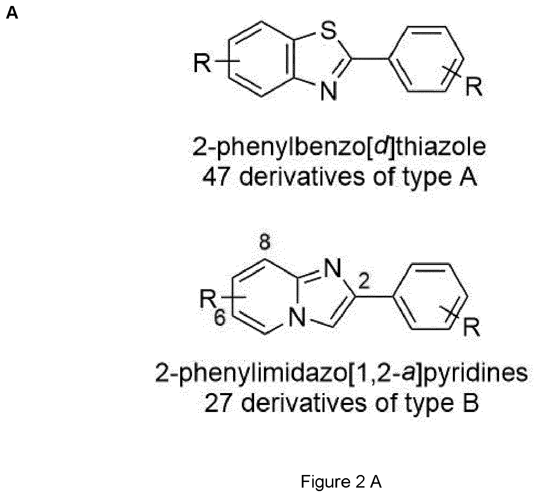

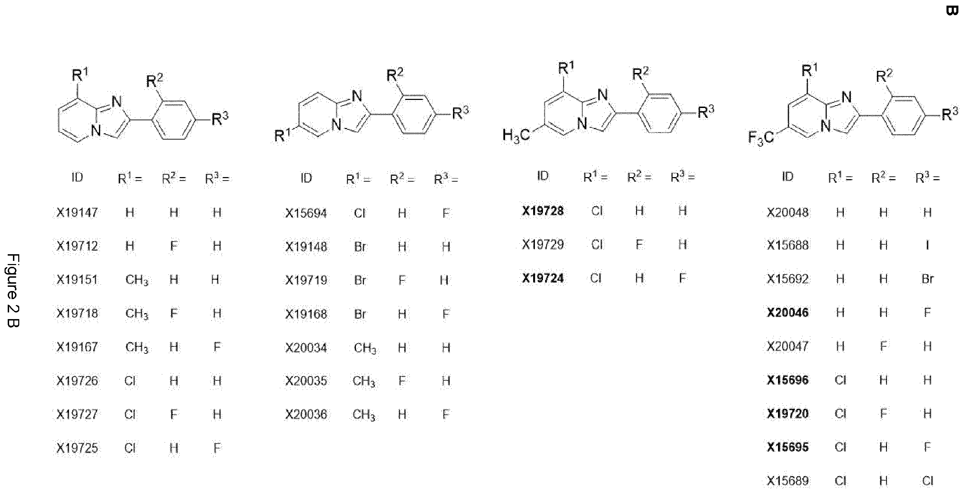

Experimental details: Single crystal X-ray diffraction data were collected on a STOE STADI VARI diffractometer with monochromated Ga Kα (λ = 1.34143 Å) radiation at 150 K. Using Olex2 (1), the structures were solved with the ShelXT (2) structure solution program using Intrinsic Phasing and refined with the ShelXL (3) refinement package using Least Squares minimization. Refinement was performed with anisotropic temperature factors for all non-hydrogen atoms; hydrogen atoms were calculated on idealized positions. Crystallographic data and structure refinement information for X15696 and X19168 are presented in Figure 2 and summarized in Table 2.

**Table 2: Crystallographic data and structure refinement information for X15696 and X19168.**

| Compound | **X15696** | **X19168** |
|---|---|---|
| Empirical formula | C₁₄H₈ClF₃N₂ | C₁₃H₈N₂FBr |
| Formula weight | 296.67 | 291.12 |
| Temperature/K | 150 | 150 |
| Crystal system | monoclinic | monoclinic |
| Space group | *P*2₁/*c* | *P*2₁/*c* |
| a/Å | 10.6224(12) | 12.8788(6) |
| b/Å | 15.1386(19) | 13.6172(9) |
| c/Å | 8.1382(9) | 6.1409(3) |
| α/° | 90 | 90 |
| β/° | 108.692(8) | 94.721(4) |
| γ/° | 90 | 90 |
| Volume/Å³ | 1239.7(3) | 1073.30(10) |
| Z | 4 | 4 |
| ρ_{calc}g/cm³ | 1.590 | 1.802 |
| µ/mm⁻¹ | 1.960 | 3.535 |
| F(000) | 600.0 | 576.0 |
| Crystal size/mm³ | 0.15 × 0.13 × 0.03 | 0.14 × 0.03 × 0.02 |
| Radiation | GaKα (λ = 1.34143) | GaKα (λ = 1.34143) |
| 2Θ range for data collection/° | 7.644-124.906 | 5.99-125.04 |
| Reflections collected | 6889 | 6923 |
| independent reflections | 2902 [Rᵢₙₜ = 0.0536] | 2528 [Rᵢₙₜ = 0.0128] |
| Ind. refl. with I ≥ 2σ(I) | 2314 | 2360 |
| Data/restraints/parameters | 2902/0/181 | 2528/0/154 |
| Goodness-of-fit on F² | 1.159 | 1.073 |
| Final R indexes [I ≥ 2σ(I)] | R₁ = 0.0960, | R₁ = 0.0223, |
| | wR₂ = 0.2621 | wR₂ = 0.0630 |
| Final R indexes [all data] | R₁ = 0.1056, | R₁ = 0.0237, |
| | wR₂ = 0.2756 | wR₂ = 0.0636 |
| Largest diff. peak/hole / e Å⁻³ | 1.44/-0.81 | 0.28/-0.42 |
| CCDC number | 2218538 | 2218539 |

### Synthesis of imidazopyridine derivatives - 8-Chloro-6-methyl-2-phenylimidazo[1,2-a]pyridine (X19728)

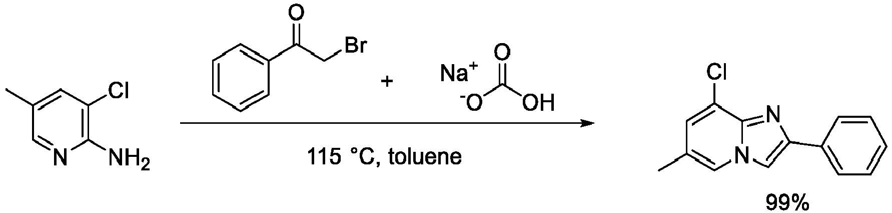

Name {X19728}: 8-chloro-6-methyl-2-phenylimidazo[1,2-a]pyridine; Formula: C₁₄H₁₁ClN₂; Exact Mass: 242.0611; Smiles: Cc1cn2cc(nc2c(c1)Cl)c1ccccc1; InChIKey: KLJOQBVCGLPIKO-UHFFFAOYSA-N

A mixture of 3-chloro-5-methyl-pyridin-2-amine (150 mg, 1.05 mmol, 1.00 equiv), 2-bromo-1-phenylethanone (209 mg, 1.05 mmol, 1.00 equiv) and sodium hydrogen carbonate (133 mg, 1.58 mmol, 1.50 equiv) in anhydrous toluene (6.00 mL) was stirred for 72 hours at 115 °C. As preparation for the column chromatography (dryload), Celite was added (0.9 g) and the reaction mixture with Celite was evaporated. The obtained crude product was purified via flash-chromatography (Interchim devices puriFLASH 5.125) on silica gel (PF-15SIHP-F0040) using cyclohexane/ethyl acetate 0% to 25% ethyl acetate in 15 column volumes (1 column volume = 91.9 mL; flow: 26 mL/min). The isolated product 8-chloro-6-methyl-2-phenylimidazo[1,2-a]pyridine (253 mg, 1.04 mmol, 99% yield) was obtained as a light orange solid in 99% yield. *R_{f}* = 0.50 (cyclohexane/ethyl acetate 2:1).

¹H NMR (400 MHz, Chloroform-d [7.27 ppm], ppm) δ = 7.98-7.95 (m, 2H), 7.79-7.78 (m, 1H), 7.71 (bs, 1H), 7.44-7.40 (m, 2H), 7.35-7.31 (m, 1H), 7.08 (d, *J* = 1.3 Hz, 1H), 2.28 (d, *J* = 1.0 Hz, 3H); ¹³C NMR (100 MHz, Chloroform-d [77.0 ppm], ppm) δ = 146.0 (C_{q}), 142.0 (C_{q}), 133.3 (C_{q}), 128.6 (2C, CH), 128.0 (CH), 126.5 (CH), 126.2 (2C, CH), 122.4 (C_{q}), 122.1 (CH), 121.9 (C_{q}), 109.4 (CH), 17.9 (CH₃); MS (EI, 70 eV, 40 °C), m/z (%): 242/244 (100/31) [M]⁺. HRMS-EI (C₁₄H₁₁N₂Cl) (*m*/*z*): [M]⁺ Calcd 242.0605; Found 242.0605; IR (ATR, ṽ) = 3128 (w), 3041 (w), 2915 (w), 1720 (w), 1686 (vw), 1636 (w), 1604 (w), 1524 (w), 1473 (m), 1443 (w), 1415 (m), 1340 (m), 1303 (w), 1283 (w), 1264 (w), 1217 (m), 1210 (m), 1149 (w), 1082 (w), 1068 (w), 1020 (w), 943 (w), 918 (w), 901 (m), 846 (w), 823 (vs), 778 (s), 722 (vs), 691 (vs), 637 (w), 616 (w), 565 (m), 558 (w), 528 (s) cm⁻¹.

### Synthesis of imidazopyridine derivatives - 8-Chloro-2-(4-fluorophenyl)-6-methylimidazo[1,2-a]pyridine (X19724)

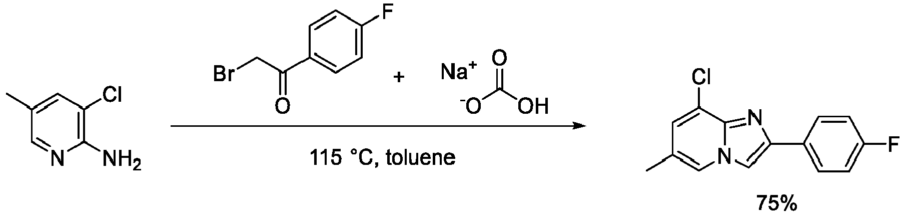

Name {X19724}: 8-chloro-2-(4-fluorophenyl)-6-methylimidazo[1,2-a]pyridine; Formula: C₁₄H₁₀ClFN₂; Exact Mass: 260.0517; Smiles: Fc1ccc(cc1)c1cn2c(n1)c(Cl)cc(c2)C; InChIKey: CFZOSNJUTXJGRX-UHFFFAOYSA-N

A mixture of 3-chloro-5-methyl-pyridin-2-amine (150 mg, 1.05 mmol, 1.00 equiv), 2-bromo-1-(4-fluorophenyl)ethanone (228 mg, 1.05 mmol, 1.00 equiv) and sodium hydrogen carbonate (133 mg, 1.58 mmol, 1.50 equiv) in anhydrous toluene (6.00 mL) was stirred for 72 hours at 115 °C. As preparation for the column chromatography (dry load), Celite was added (0.9 g) and the reaction mixture with Celite was evaporated. The obtained crude product was purified via flash-chromatography (Interchim devices puriFLASH 5.125) on silica gel (PF-15SIHP-F0040) using cyclohexane/ethyl acetate 0% to 25% ethyl acetate in 15 column volumes (1 column volume = 91.9 mL; flow: 26 mL/min). The isolated product 8-chloro-2-(4-fluorophenyl)-6-methylimidazo[1,2-a]pyridine (207 mg, 794 µmol) was obtained as a light orange solid in 75% yield. *R_{f}* = 0.46 (cyclohexane/ethyl acetate 2:1).

¹H NMR (400 MHz, Chloroform-d [7.27 ppm], ppm) δ = 7.96-7.91 (m, 2H), 7.82 (t, *J =* 1.2 Hz, 1H), 7.75 (s, 1H), 7.14-7.08 (m, 3H), 2.31 (d, *J =* 1.0 Hz, 3H); ¹³C NMR (100 MHz, Chloroform-d [77.0 ppm], ppm) δ = 162.8 (d, *J* = 246.6 Hz, C_{q}), 145.2 (C_{q}), 142.0 (C_{q}), 129.5 (d, *J* = 3.1 Hz, C_{q}), 127.9 (d, *J* = 8.5 Hz, CH, 2C), 126.7 (CH), 122.4 (C_{q}), 122.1 (C_{q}), 122.1 (CH), 115.5 (d, *J* = 21.6 Hz, CH, 2C), 109.1 (CH), 17.9 (CH₃); ¹⁹F NMR (376 MHz, ppm) δ = -113.91; MS (EI, 70 eV, 90 °C), m/z (%): 260/262 (100/31) [M]⁺. HRMS-EI (C₁₄H₁₀N₂ClF) (*m*/*z*): [M]⁺ Calcd 260.0511; Found 260.0510; IR (ATR, ṽ) = 3131 (w), 3080 (w), 3054 (w), 3040 (w), 2953 (w), 2919 (w), 1596 (w), 1548 (w), 1526 (m), 1499 (w), 1477 (s), 1449 (m), 1411 (s), 1377 (w), 1356 (w), 1343 (m), 1293 (w), 1282 (w), 1271 (w), 1211 (vs), 1157 (s), 1123 (w), 1099 (s), 1078 (m), 1041 (w), 1021 (m), 989 (w), 969 (w), 943 (w), 902 (s), 881 (m), 867 (m), 854 (vs), 823 (vs), 805 (vs), 738 (vs), 703 (vs), 674 (m), 662 (s), 633 (m), 605 (m), 569 (m), 543 (s), 531 (vs), 513 (s) cm⁻¹.

### Synthesis of imidazopyridine derivatives - 8-Chloro-2-phenyl-6-(trifluoromethyl)imidazo[1,2-a]pyridine (X19166/X15696)

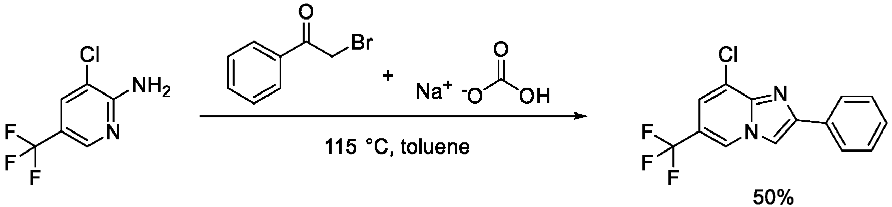

Name {X19166/X15696}: 8-chloro-2-phenyl-6-(trifluoromethyl)imidazo[1,2-a]pyridine; Formula: C₁₄HaClF₃N₂; Exact Mass: 296.0328; Smiles: Clc1cc(cn2c1nc(c2)c1ccccc1)C(F)(F)F; InChIKey: ATHNSQRFCXRPMB-UHFFFAOYSA-N

A mixture of 3-chloro-5-(trifluoromethyl)pyridin-2-amine (150 mg, 763 µmol, 1.00 equiv), 2-bromo-1-phenylethanone (152 mg, 763 µmol, 1.00 equiv) and sodium hydrogen carbonate (96.2 mg, 1.14 mmol, 1.50 equiv) in anhydrous toluene (4.00 mL) was stirred for 16 hours at 115 °C. As preparation for the column chromatography (dryload), Celite was added (0.9 g) and the reaction mixture with Celite was evaporated. The obtained crude product was purified via flash-chromatography (Interchim devices puriFLASH 5.125) on silica gel (PF-15SIHP-F0040) using cyclohexane/ethyl acetate 0% to 10% ethyl acetate in 15 column volumes (1 column volume = 91.9 mL; flow: 26 mL/min). The isolated product 8-chloro-2-phenyl-6-(trifluoromethyl)imidazo[1,2-a]pyridine (112 mg, 379 µmol) was obtained as a colorless solid in 50% yield. *R_{f}* = 0.73 (cyclohexane/ethyl acetate 2:1).

¹H NMR (400 MHz, Chloroform-d [7.27 ppm], ppm) δ = 8.43 (t, *J* = 1.2 Hz, 1H), 8.01-7.98 (m, 3H), 7.48-7.41 (m, 2H), 7.42-7.34 (m, 2H); ¹³C NMR (100 MHz, Chloroform-d [77.0 ppm], ppm) δ = 148.3 (C_{q}), 142.8 (C_{q}), 132.3 (C_{q}), 128.9 (2C, CH), 128.8 (CH), 126.5 (2C, CH), 124.3 (C_{q}), 123.1 (q, *J* = 5.8 Hz, CH), 122.9 (q, *J* = 271.3 Hz, CF₃), 119.5 (q, *J* = 3.1 Hz, CH), 116.7 (q, *J* = 34.7 Hz, C_{q}), 110.8 (CH); ¹⁹F NMR (376 MHz, ppm) δ = -62.02; MS (EI, 70 eV, 30 °C), m/z (%): 332 (16), 330 (25), 296/298 (100/32) [M]+, 196/198 (58/20), 141 (11), 116 (16), 105 (97), 89 (10), 77 (46), 71 (11), 58 (37), 51 (15); HRMS-EI (C₁₄H₈N₂ClF₃) *(m*/*z):* [M]⁺ Calcd 296.0323; Found 296.0325; IR (ATR, ṽ) = 3122 (w), 3092 (w), 3057 (w), 2924 (w), 2853 (vw), 1714 (w), 1701 (w), 1642 (w), 1598 (w), 1541 (vw), 1476 (w), 1449 (w), 1434 (w), 1377 (m), 1344 (s), 1332 (m), 1316 (vs), 1272 (m), 1237 (w), 1224 (w), 1191 (m), 1171 (vs), 1118 (vs), 1072 (vs), 1027 (m), 992 (s), 958 (m), 933 (m), 922 (w), 890 (s), 857 (vs), 815 (m), 781 (s), 758 (w), 747 (w), 727 (vs), 710 (vs), 691 (vs), 680 (s), 646 (s), 640 (m), 616 (m), 579 (w), 567 (w), 544 (w), 535 (w), 524 (m) cm⁻¹.

### Synthesis of imidazopyridine derivatives - 8-Chloro-2-(2-fluorophenyl)-6-(trifluoromethyl)imidazo[1,2-a]pyridine (X19720)

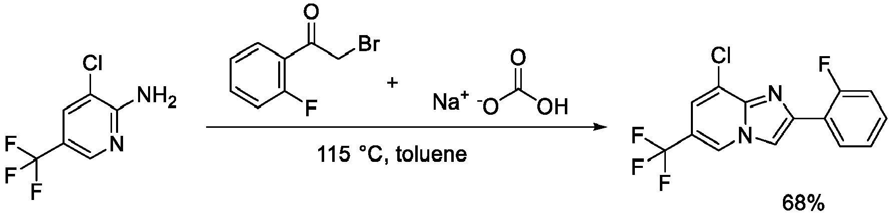

Name {X19720}: 8-chloro-2-(2-fluorophenyl)-6-(trifluoromethyl)imidazo[1,2-a]pyridine; Formula: C₁₄H₇ClF₄N₂; Exact Mass: 314.0234; Smiles: Fc1ccccc1c1cn2c(n1)c(Cl)cc(c2)C(F)(F)F; InChIKey: RAEZJUWRAYPJAR-UHFFFAOYSA-N

A mixture of 3-chloro-5-(trifluoromethyl)pyridin-2-amine (150 mg, 763 µmol, 1.00 equiv), 2-bromo-1-(2-fluorophenyl)ethanone (166 mg, 763 µmol, 1.00 equiv) and sodium hydrogen carbonate (96.2 mg, 1.14 mmol, 1.50 equiv) in anhydrous toluene (4.00 mL) was stirred for 16 hours at 115 °C. As preparation for the column chromatography (dryload), Celite was added (0.9 g) and the reaction mixture with Celite was evaporated. The obtained crude product was purified via flash-chromatography (Interchim devices puriFLASH 5.125) on silica gel (PF-15SIHP-F0040) using cyclohexane/ethyl acetate 0% to 10% ethyl acetate in 15 column volumes (1 column volume = 91.9 mL; flow: 26 mL/min). The isolated product 8-chloro-2-(2-fluorophenyl)-6-(trifluoromethyl)imidazo[1,2-a]pyridine (164 mg, 520 µmol) was obtained as a colorless solid in 68% yield. *R_{f}* = 0.75 (cyclohexane/ethyl acetate 2:1).

¹H NMR (400 MHz, Chloroform-d [7.27 ppm], ppm) δ = 8.47-8.43 (m, 2H), 8.19 (d, *J* = 3.7 Hz, 1H), 7.42 (d, *J* = 1.5 Hz, 1H), 7.39-7.33 (m, 1H), 7.31-7.27 (m, 1H), 7.16 (ddd, *J* = 1.2 Hz, *J* = 11.5 Hz, *J* = 8.1 Hz, 1H); ¹³C NMR (100 MHz, Chloroform-d [77.0 ppm], ppm) δ = 160.4 (d, *J* = 249.7 Hz, C_{q}), 142.0 (C_{q}), 141.6 (d, *J* = 1.5 Hz, C_{q}), 130.0 (d, *J* = 8.5 Hz, CH), 129.4 (d, *J* = 3.1 Hz, CH), 124.6 (d, *J* = 3.1 Hz, CH), 124.3 (C_{q}), 123.3 (q, *J* = 6.2 Hz, CH), 122.9 (q, *J =* 271.5 Hz, CF₃), 120.3 (d, *J* = 11.6 Hz, C_{q}), 119.7 (q, *J* = 2.7 Hz, CH), 116.7 (q, *J* = 34.9 Hz, C_{q}), 115.6 (d, *J =* 22.3 Hz, CH), 114.6 (d, *J* = 15.4 Hz, CH); ¹⁹F NMR (376 MHz, ppm) δ = -62.07 (s), -113.69--113.76 (m); MS (EI, 70 eV, 50 °C), m/z (%): 314/316 (100/31) [M]⁺, 134 (11); HRMS-EI (C₁₄H₇N₂ClF₄) *(m*/*z)*: [M]⁺ Calcd 314.0228; Found 314.0227; IR (ATR, ṽ) = 3166 (w), 3111 (w), 1728 (vw), 1642 (w), 1581 (w), 1547 (w), 1482 (m), 1448 (w), 1431 (vw), 1384 (m), 1377 (m), 1350 (s), 1330 (vs), 1309 (vs), 1255 (w), 1239 (w), 1222 (m), 1205 (m), 1190 (s), 1174 (s), 1164 (s), 1152 (s), 1125 (vs), 1071 (vs), 1028 (m), 994 (s), 945 (w), 936 (w), 892 (s), 870 (s), 858 (vs), 827 (w), 800 (s), 771 (vs), 749 (vs), 741 (vs), 717 (vs), 703 (vs), 673 (s), 647 (w), 639 (w), 592 (w), 579 (w), 565 (m), 531 (m), 516 (m) cm⁻¹.

### Synthesis of imidazopyridine derivatives - 8-Chloro-2-(4-fluorophenyl)-6-(trifluoromethyl)imidazo[1,2-a]pyridine (X15695)

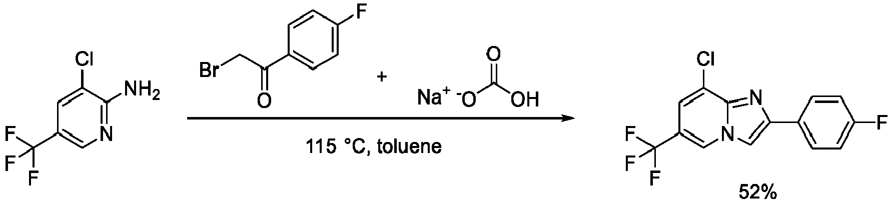

Name {X15695}: 8-chloro-2-(4-fluorophenyl)-6-(trifluoromethyl)imidazo[1,2-a]pyridine; Formula: C₁₄H₇ClF₄N₂; Exact Mass: 314.0234; Smiles: Fc1ccc(cc1)c1cn2c(n1)c(Cl)cc(c2)C(F)(F)F; InChIKey: LHUTUFIEQFAVFB-UHFFFAOYSA-N

A mixture of 3-chloro-5-(trifluoromethyl)pyridin-2-amine (500 mg, 2.54 mmol, 1.00 equiv), 2-bromo-1-(4-fluorophenyl)ethanone (524 mg, 2.42 mmol, 0.950 equiv) and sodium hydrogen carbonate (321 mg, 3.82 mmol, 1.50 equiv) in anhydrous toluene (15.00 mL) was stirred for 4 days at 115 °C. As preparation for the column chromatography (dryload), Celite was added (1.50 g) and the reaction mixture with Celite were evaporated. The obtained crude product was purified via flash-chromatography (Interchim devices puriFLASH 5.125) on silica gel (PF-15SIHP-F0080) using cyclohexane/ethyl acetate 0% to 10% ethyl acetate in 15 column volumes (1 column volume = 173.2 mL; flow: 34 mL/min). The isolated product 8-chloro-2-(4-fluorophenyl)-6-(trifluoromethyl)imidazo[1,2-a]pyridine (416 mg, 1.32 mmol) was obtained as a colorless solid in 52% yield. *R_{f}* = 0.71 (cyclohexane/ethyl acetate 2:1).

¹H NMR (400 MHz, Chloroform-d [7.27 ppm], ppm) δ = 8.43 (pseudo-t, *J* = 1.2 Hz, 1H), 7.99-7.94 (m, 2H), 7.94 (s, 1H), 7.42 (d, *J =* 1.6 Hz, 1H), 7.17-7.13 (m, 2H); ¹³C NMR (100 MHz, Chloroform-d [77.0 ppm], ppm) δ = 163.2 (d, *J* = 248.1 Hz, C_{q}), 147.4 (C_{q}), 142.8 (C_{q}), 128.6 (d, *J* = 3.1 Hz, C_{q}), 128.3 (d, *J* = 8.5 Hz, 2C, CH), 124.3 (C_{q}), 123.1 (q, *J* = 5.4 Hz, CH), 122.9 (q, *J* = 271.3 Hz, CF₃), 119.6 (q, *J =* 3.1 Hz, CH), 116.8 (q, *J* = 35.1 Hz, C_{q}), 115.8 (d, *J =* 22.3 Hz, 2C, CH), 110.4 (CH); ¹⁹F NMR (376 MHz, ppm) δ = -62.02, -112.47; MS (EI, 70 eV, 60 °C), m/z (%): 314/316 (100/33) [M]⁺, HRMS-EI (C₁₄H₇N₂ClF₄) *(m*/*z)*: [M]⁺ Calcd 314.0228; Found 314.0227; IR (ATR, ṽ) = 3126 (w), 3092 (w), 3060 (w), 1723 (w), 1642 (w), 1601 (w), 1548 (w), 1486 (s), 1456 (w), 1432 (w), 1417 (w), 1375 (m), 1349 (s), 1329 (vs), 1307 (vs), 1272 (w), 1237 (w), 1218 (vs), 1193 (s), 1170 (vs), 1156 (vs), 1125 (vs), 1101 (vs), 1074 (vs), 1013 (m), 994 (s), 946 (w), 935 (m), 891 (s), 863 (m), 850 (vs), 823 (s), 803 (vs), 742 (vs), 698 (vs), 676 (s), 666 (m), 647 (m), 632 (m), 602 (m), 568 (m), 541 (s), 527 (s), 511 (m) cm⁻¹.

### Synthesis of imidazopyridine derivatives - 2-(4-Fluorophenyl)-6-(trifluoromethyl)imidazo[1,2-a]pyridine (X20046)

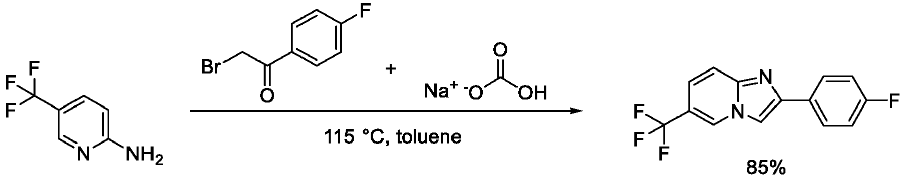

Name {X20046}: 2-(4-fluorophenyl)-6-(trifluoromethyl)imidazo[1,2-a]pyridine; Formula: C₁₄H₈F₄N₂; Exact Mass: 280.0624; Smiles: Fc1ccc(cc1)c1cn2c(n1)ccc(c2)C(F)(F)F; InChIKey: RFCPFCDGJGHFCX-UHFFFAOYSA-N

A mixture of 5-(trifluoromethyl)pyridin-2-amine (200 mg, 1.23 mmol, 1.00 equiv), 2-bromo-1-(4-fluorophenyl)ethanone (268 mg, 1.23 mmol, 1.00 equiv) and sodium hydrogen carbonate (155 mg, 1.85 mmol, 1.50 equiv) in anhydrous toluene (6.00 mL) was stirred for 16 hours at 115 °C. As preparation for the column chromatography (dryload), Celite was added (0.6 g) and the reaction mixture with Celite were evaporated. The obtained crude product was purified via flash-chromatography (Interchim devices puriFLASH 5.125) on silica gel (PF-15SIHP-F0040) using cyclohexane/ethyl acetate 0% to 20% ethyl acetate in 20 column volumes (1 column volume = 91.9 mL; flow: 26 mL/min). The isolated product 2-(4-fluorophenyl)-6-(trifluoromethyl)imidazo[1,2-a]pyridine (293 mg, 1.04 mmol) was obtained as a light-yellow solid in 85% yield. *R_{f}* = 0.57 (cyclohexane/ethyl acetate 2:1).

¹H NMR (400 MHz, Chloroform-d [7.27 ppm], ppm) δ = 8.49-8.48 (m, 1H), 7.96-7.91 (m, 2H), 7.89 (s, 1H), 7.72 (dd, *J* = 0.6 Hz, *J* = 9.4 Hz, 1H), 7.33 (dd, *J =* 1.8 Hz, *J* = 9.5 Hz, 1H), 7.18-7.12 (m, 2H); ¹³C NMR (100 MHz, Chloroform-d [77.0 ppm], ppm) δ = 163.0 (d, *J* = 248.1 Hz, C_{q}), 146.9 (C_{q}), 145.3 (C_{q}), 129.1 (d, *J* = 3.9 Hz, C_{q}), 127.9 (d, *J* = 8.5 Hz, 2C, CH), 124.5 (q, *J* = 5.8 Hz, CH), 123.5 (q, *J =* 271.0 Hz, CF₃), 120.7 (q, *J* = 2.6 Hz, CH), 118.0 (CH), 117.0 (q, *J* = 34.2 Hz, C_{q}), 115.8 (d, *J* = 21.6 Hz, 2C, CH), 108.8 (CH); ¹⁹F NMR (376 MHz, ppm) δ = -62.15 (CF3), -112.89 (CF); MS (EI, 70 eV, 40 °C), m/z (%): 280 (100) [M]⁺, HRMS-EI (C₁₄H₈N₂F₄) *(m*/*z)*: [M]⁺ Calcd 280.0618; Found 280.0619; IR (ATR, ṽ) = 1647 (w), 1611 (w), 1599 (w), 1550 (vw), 1489 (s), 1441 (w), 1417 (vw), 1383 (w), 1334 (vs), 1312 (vs), 1271 (w), 1234 (s), 1225 (s), 1200 (w), 1167 (vs), 1119 (vs), 1052 (vs), 1014 (w), 970 (w), 939 (m), 870 (m), 846 (vs), 822 (m), 806 (vs), 759 (m), 744 (vs), 708 (m), 670 (vs), 640 (s), 582 (w), 565 (w), 535 (m), 516 (vs) cm⁻¹.

### Synthesis of imidazopyridine derivatives - 6-Bromo-2-(4-fluorophenyl)imidazo[1,2-a]pyridine (X19168)

Name {X19168}: 6-bromo-2-(4-fluorophenyl)imidazo[1,2-a]pyridine; Formula: C₁₃H₈BrFN₂; CAS: - ; Molecular Mass: 291.1184; Exact Mass: 289.9855; EA: Br, 27.45; C, 53.63; F, 6.53; H, 2.77; N, 9.62. Smiles: Fc1ccc(cc1)c1cn2c(n1)ccc(c2)Br; InChIKey: NKIQEILZYPFNFA-UHFFFAOYSA-N

A mixture of 5-bromopyridin-2-amine (150 mg, 867 µmol, 1.00 equiv), 2-bromo-1-(4-fluorophenyl)ethanone (188 mg, 867 µmol, 1.00 equiv) and sodium hydrogen carbonate (109 mg, 1.30 mmol, 1.50 equiv) in anhydrous toluene (7.00 mL) was stirred for 16 hours at 115 °C. As preparation for the column chromatography (dryload), Celite was added (0.9 g) and the reaction mixture with Celite was evaporated. The obtained crude product was purified via flash-chromatography (Interchim devices puriFLASH 5.125) on silica gel (PF-15SIHP-F0040) using cyclohexane/ethyl acetate 0% to 30% ethyl acetate in 20 column volumes (1 column volume = 91.9 mL; flow: 26 mL/min). The isolated product 6-bromo-2-(4-fluorophenyl)imidazo[1,2-a]pyridine (147 mg, 505 µmol) was obtained as a colorless solid in 58% yield. *R_{f}* = 0.49 (cyclohexane/ethyl acetate 2:1).

¹H NMR (400 MHz, Chloroform-d [7.27 ppm], ppm) δ = 8.27 (dd, *J* = 0.9 Hz, *J* = 1.8 Hz, 1H), 7.94-7.89 (m, 2H), 7.77 (s, 1H), 7.54 (dd, *J* = 0.9 Hz, *J* = 9.4 Hz, 1H), 7.26 (dd, 1.9 Hz, *J* = 9.5 Hz, 1H), 7.16-7.10 (m, 2H); ¹³C NMR (100 MHz, Chloroform-d [77.0 ppm], ppm) δ = 162.9 (d, *J* = 247.4 Hz, C_{q}), 145.5 (C_{q}), 143.9 (C_{q}), 129.2 (d, *J* = 3.1 Hz, C_{q}), 128.4 (CH), 127.8 (d, *J* = 7.7 Hz, 2C, CH), 125.6 (CH), 117.9 (CH), 115.8 (d, *J* = 21.6 Hz, 2C, CH), 107.9 (C_{q}), 107.2 (CH); ¹⁹F NMR (376 MHz, ppm) δ = -113.27; MS (EI, 70 eV, 70 °C), m/z (%): 290/292 (100/99) [M]+, 210 (15), 123 (12), 63 (11), 58 (13). HRMS-EI (C₁₃H₈N₂BrF) *(m*/*z):* [M]⁺ Calcd 289.9849; Found 289.9848; IR (ATR, ṽ) = 3132 (vw), 1596 (w), 1544 (vw), 1519 (w), 1477 (w), 1419 (w), 1407 (w), 1334 (w), 1315 (w), 1293 (w), 1256 (w), 1232 (w), 1214 (w), 1200 (w), 1150 (w), 1137 (w), 1095 (m), 1077 (w), 1052 (m), 1007 (w), 960 (w), 946 (w), 936 (w), 926 (w), 841 (s), 832 (vs), 795 (vs), 737 (vs), 707 (vs), 686 (m), 676 (m), 637 (m), 598 (w), 568 (m), 518 (vs) cm⁻¹.

### Graphing and statistical analysis

Experiments were performed with three or more replicates. Differences between two groups were analyzed by Student's t-test and multiple comparisons were determined by one-way ANOVA. If there were two factors (such as dose and time) investigated, data were analyzed by two-way ANOVA followed by a post-hoc test. Data were expressed as means ± SEM, and P < 0.05 was considered significant. All analyses were performed using Microsoft Excel 2010 and GraphPad Prism 8.3.1 software.

### Example 1:

### Growth inhibitory properties of imidazopyridine derivatives

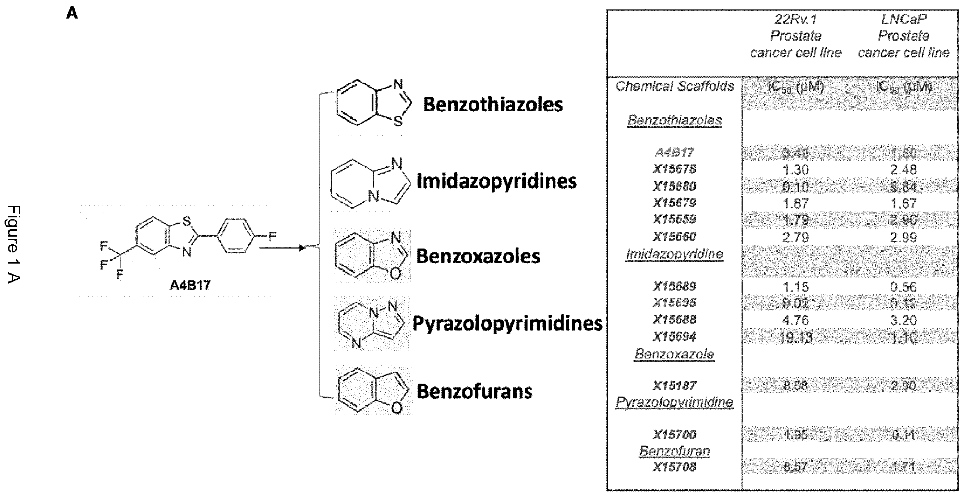

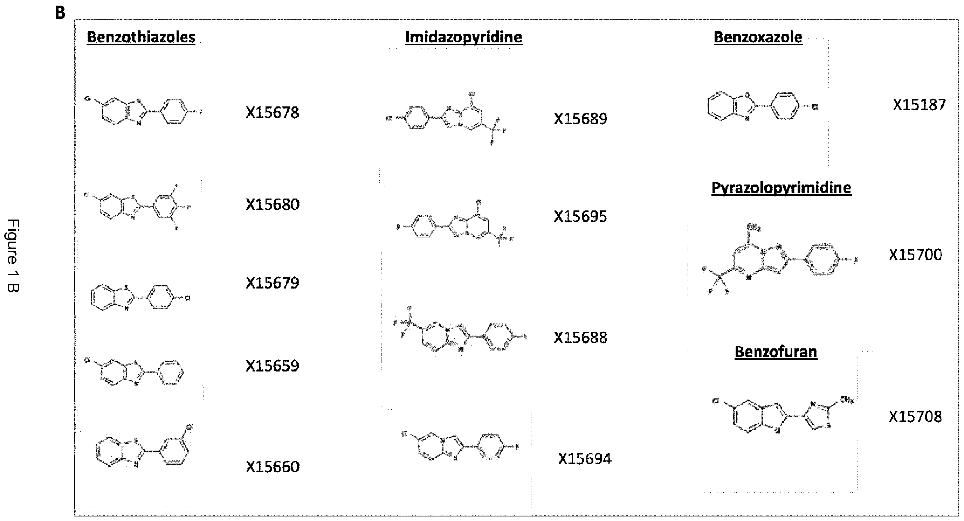

It has previously been reported that the BAG1-selective compound A4B17 derived from a screen of 47 benzothiazoles inhibits proliferation of ER⁺ and AR⁺ breast and prostate cancer cells with IC₅₀ values in the micromolar range. To improve the potency of this compound, a scaffold hopping approach was used focusing on heterocycle replacement (Fig. 1A-B). This identified the imidazopyridine X15695 as the most potent compound in the inhibition of the clonal expansion of the prostate cancer cell lines LNCaP and 22Rv.1 (Fig. 1A). The benzothiazole scaffold was therefore change into an imidazopyridine (Fig. 2A) and twenty-seven imidazopyridine derivatives were synthesized (Fig. 2B) and analyzed. This greatly improved the inhibitory properties of the imidazopyridine derivatives both in breast and prostate cancer cells (Table 1 A and B).

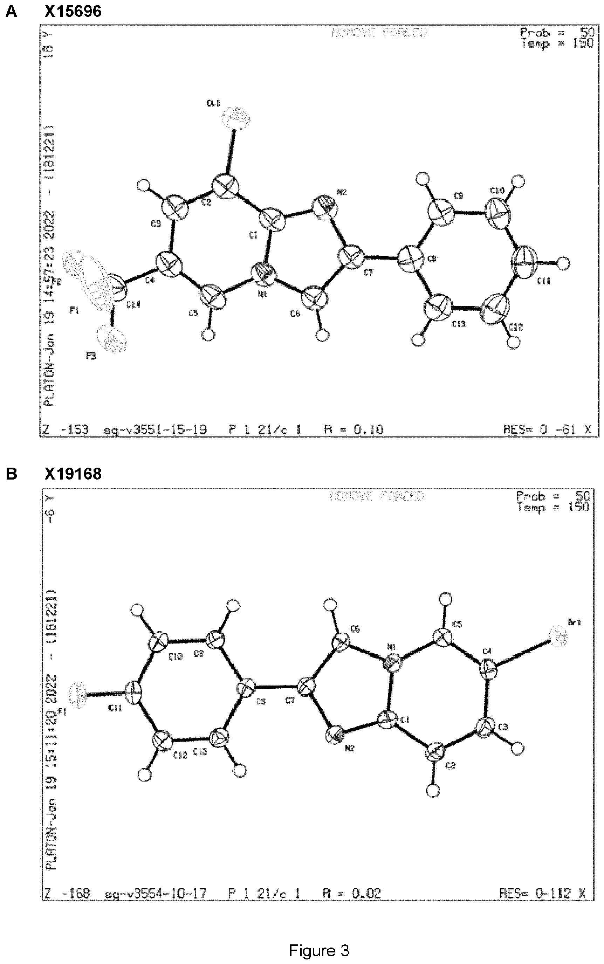

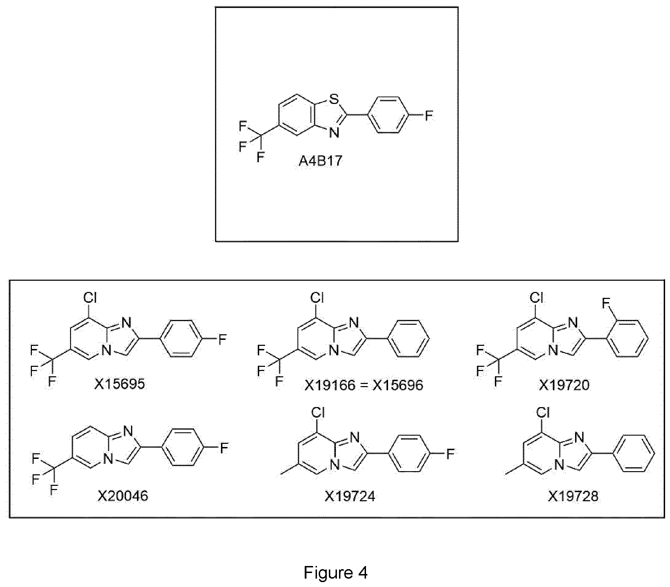

The X-ray structure of compounds X15696 and X19168 (Fig. 3; Table 1 A and B) was determined for rational drug design. All the compounds outperformed A4B17 in the inhibition of clonal expansion of ER⁺ and AR⁺ breast and prostate cancer cells and had a very weak or no noticeable effect on receptor-negative cell lines (Table 1 A and B). On the whole, compounds that potently inhibited colony formation of ER+ cells also potently inhibited the clonal expansion of AR+ prostate cell lines. Only very few compounds showed differences in potencies towards ER+ and AR+ cell lines. Six imidazopyridines, including X20046 (the imidazopyridine derivative of A4B17) were identified as particularly potent in their inhibitory profiles based on their IC₅₀ values in the two different cell types (Table 1 A and B). Structurally, these six compounds share a striking resemblance in their core structures (Fig. 4) and show a requirement for either a methyl- or a CF₃-group at position C-6 of the imidazopyridine core similar to other derivatives that were only active against one of the two cancer cell types analyzed. From the six identified compounds, X15695 was chosen for further study.

**Table 1 A: Inhibitory properties of imidazopyridine derivatives (breast cancer)**

| **Compound** | **Breast Cancer (MCF-7)** | **Breast Cancer (ZR75-1)** | **Breast Cancer (T47D)** | **Breast Cancer (MDA-MB231)** |
|---|---|---|---|---|
| | **ER+** | **ER+** | **ER+** | **ER-** |
| | IC₅₀ (nM) | IC₅₀ (nM) | IC₅₀ (nM) | IC₅₀ (nM) |
| **A4B17** | 1280 | 110 | | n.d |
| X19729 | 20.8 | | | |
| **X19728** | 6.0±2.8 | 39.2±24.3 | 153.3±67. 9 | n.d |
| **X19724** | 3.3±0.9 | 16.2±4.7 | 86.1±49.1 | n.d |
| X19727 | 19.2 | | | |
| X19726 | 84.6 | | | |
| X19725 | 66.9 | | | |
| X19712 | 2846 | | | |
| X19718 | 720.2 | | | |
| X19719 | 12.9 | 19.3 | 100 | |
| **X19720** | 14.5 | | | |
| X19168 | 12.0 | | | |
| X19167 | 486.2 | | | |
| **X19166/ X15696** | 7.9±1.9 | 10.2±2.7 | 44.7±11.4 | n.d |
| X19148 | 228.3 | | | |
| X19151 | 135.6 | | | |
| X19147 | 18.3 | | | |
| **X15695** | 3.5±1.7 | 7.3±1.6 | 10.4±2.8 | 5540 |
| X15689 | 24.1 | | | |
| X15688 | 213.3 | | | |
| X15694 | 182.7 | | | |
| X15692 | 54.4 | | | |
| X20034 | n.d | | | |
| X20035 | 1570 | | | |
| X20036 | n.d | | | |
| **X20046** | 10.0±4.3 | 31.8 | 57.1 | n.d |
| X20047 | 57.8 | | | |
| X20048 | 589.4 | | | |

**Table 1 B: Inhibitory properties of imidazopyridine derivatives (prostate cancer)**

| **Compound** | **Prostate Cancer (22Rv.1)** | **Prostate Cancer (LNCaP)** | **Prostate Cancer (PC3)** | **Prostate Cancer (DU145)** |
|---|---|---|---|---|
| | **AR+** | **AR+** | **AR-** | **AR-** |
| | IC₅₀ (nM) | IC₅₀ (nM) | IC₅₀ (nM) | IC₅₀ (nM) |
| **A4B17** | 3400 | 1600 | n.d | n.d |
| **X19729** | 140 | | | |
| **X19728** | 48.5±13.3 | n.d | | n.d. |
| **X19724** | 4.4 | 0.2 | | n.d |
| **X19727** | 280 | | | |
| **X19726** | n.d | | | |
| **X19725** | 651±168 | < 0.1 | | |
| **X19712** | 2790 | | | |
| **X19718** | 3480 | | | |
| **X19719** | 530 | | | |
| **X19720** | 4.4±1.6 | < 0.1 | | n.d. |
| **X19168** | 641.0±246.3 | | | |
| **X19167** | 1880 | | | |
| **X19166/ X15696** | 22.5±6.6 | < 0.1 | 11930 | 3581 |
| **X19148** | 2410 | | | |
| **X19151** | n.d. | | | |
| **X19147** | n.d. | | | |
| **X15695** | 40.2±16.7 | 14.1 | 2040 | 1340 |
| **X15689** | 1150 | 560 | 2370 | 3170 |
| **X15688** | 4760 | 3200 | | |
| **X15694** | 10130 | 1100 | | |
| **X15692** | 520 | 1170 | n.d. | 15090 |
| **X20034** | n.d, 9500 | | | |
| **X20035** | n.d | | | |
| **X20036** | n.d | | | |
| **X20046** | 7.5±3.2 | 0.1 | n.d | n.d |
| **X20047** | 135.5 | | | |
| **X20048** | 561.8 | | | |

### Example 2

### Comparison of activity of selected imidazopyridines with currently available AR antagonists.

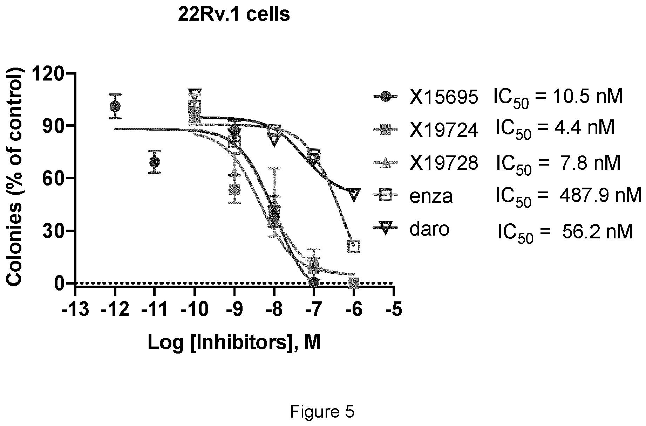

Three out of the selected six imidazopyridines containing X15695 were compared with two currently available and clinically used AR antagonists enzalutamide (enza) and darolutamide (daro) for their ability to inhibit proliferation of the CRPC cell line 22Rv.1. All three imidazopyridines had roughly similar IC₅₀ values in inhibiting clonal expansion of the 22Rv.1 cells but these differed significantly from the inhibitory action of enzalutamide and darolutamide (Fig. 5). These data demonstrate a favorable inhibitory profile of the imidazopyridine compounds for CRPC cells.

### Example 3:

### Transcriptomics analyses to determine X15695 action

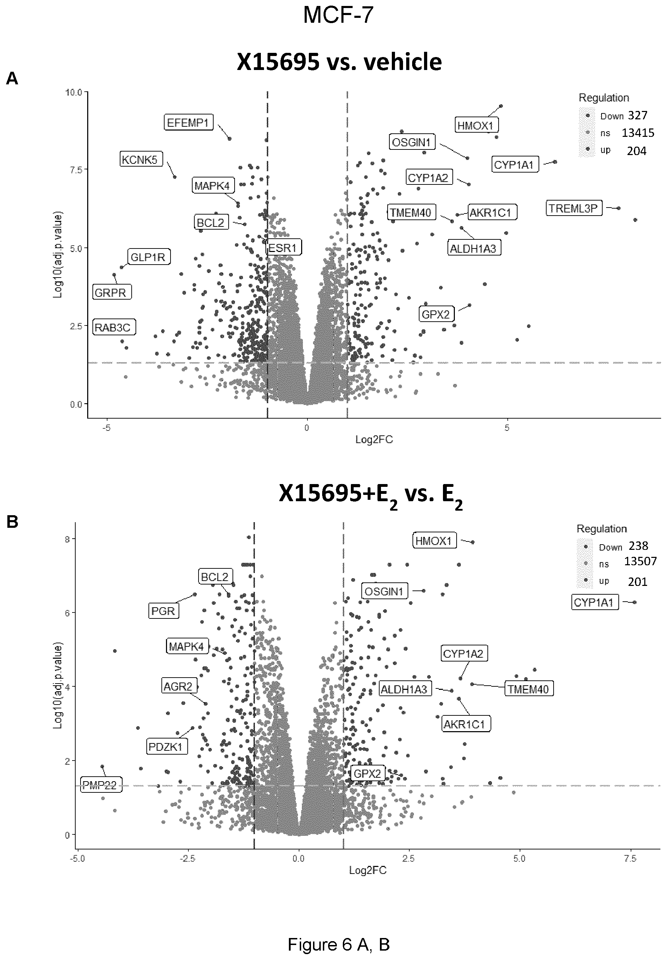

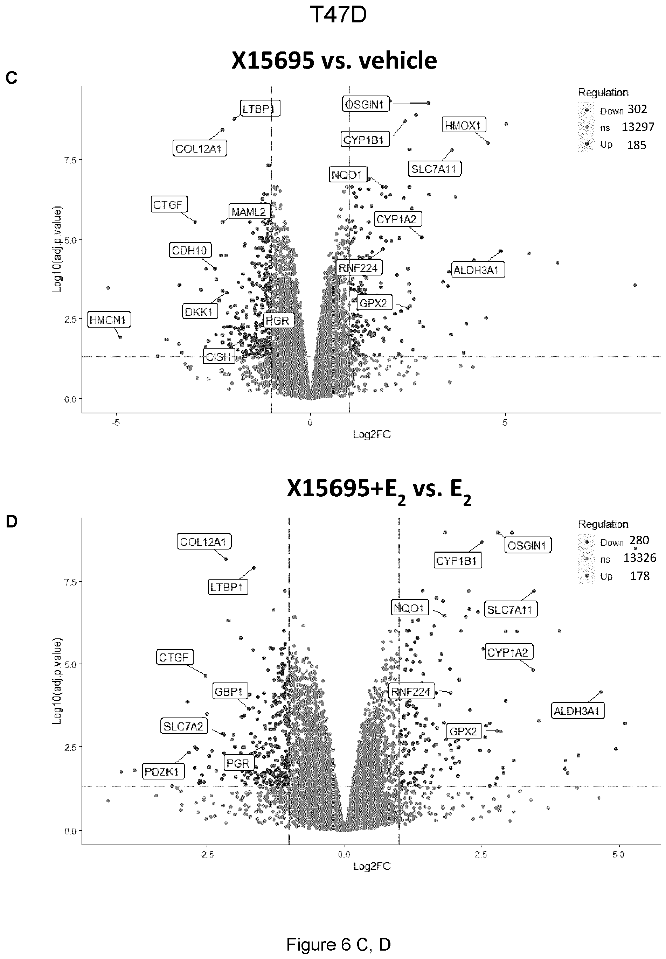

To determine the mechanism of action of X15695, at first transcriptomics analyses were performed in ER⁺ breast cancer cells MCF-7 and T47D after treating them with vehicle (DMSO), 17-β-estradiol (E₂), X15695 and a combination of E₂ and X15695. The RNA-seq datasets were analyzed using a fold change of Log₂(CPM) ≥ 1.0 or ≤ -1.0 and an adj. p value ≤ 0.05 to identify differentially expressed genes (DEGs) in response to X15695 in the presence or absence of E₂. A total of 531 DEGs (327 DEGs downregulated and 204 DEGs upregulated) was identified in MCF-7 cells in the absence of E₂ but 439 DEGs (238 DEGs downregulated and 201 DEGs upregulated) in the presence of E₂ (Fig. 6 A, B). In the T47D cells, 487 DEGs (302 downregulated and 185 upregulated) were identified in response to X15695 in the absence of E₂ treatment while in the presence of E₂, slightly less DEGs were obtained (458; 280 downregulated and 176 genes upregulated) (Fig. 6 C, D).

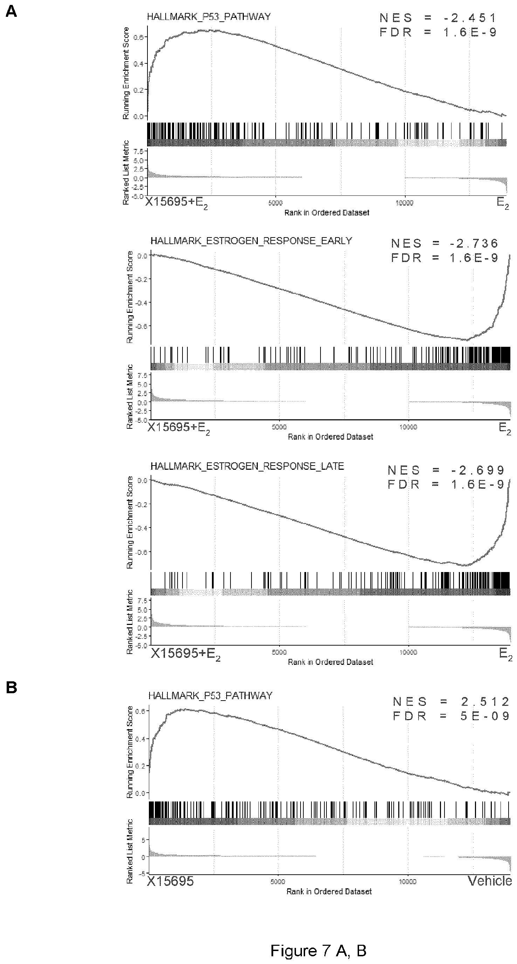

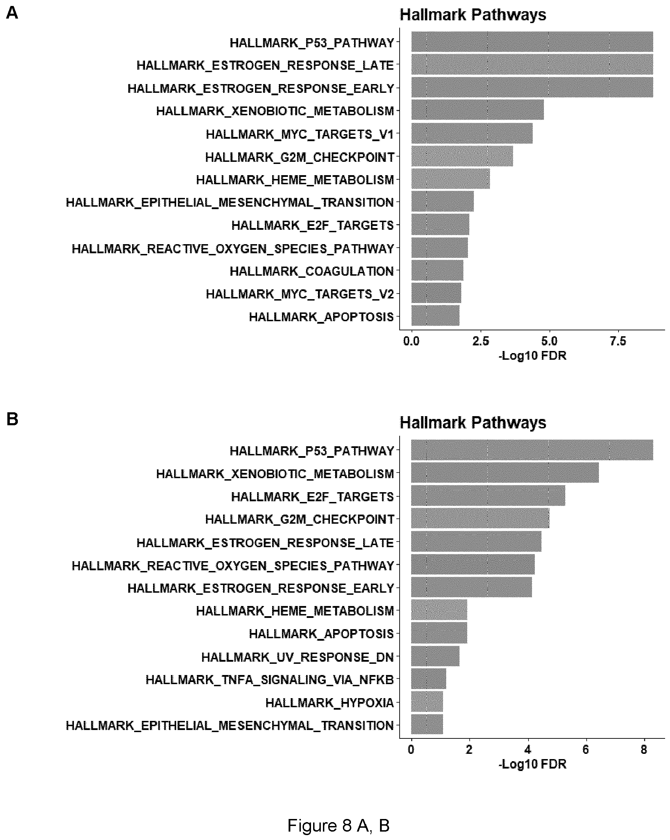

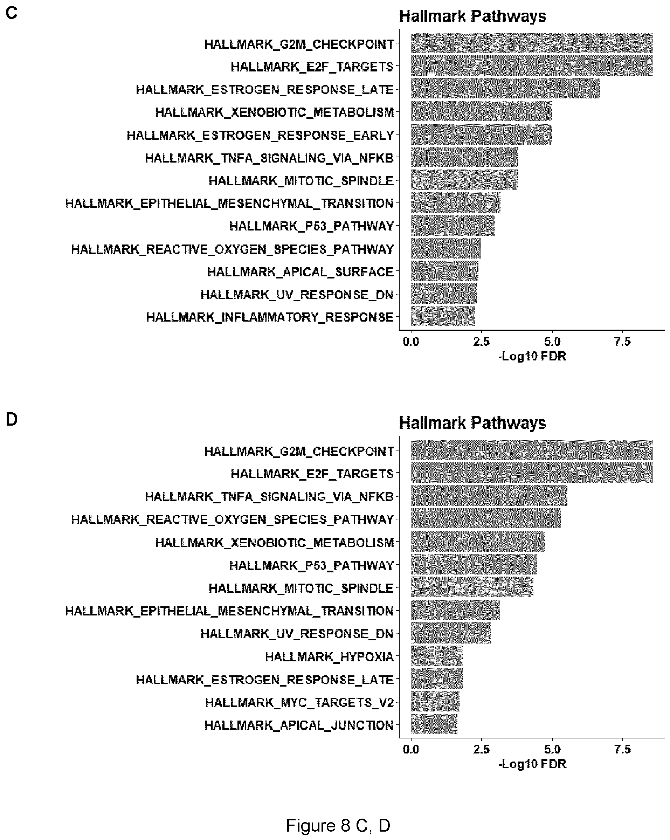

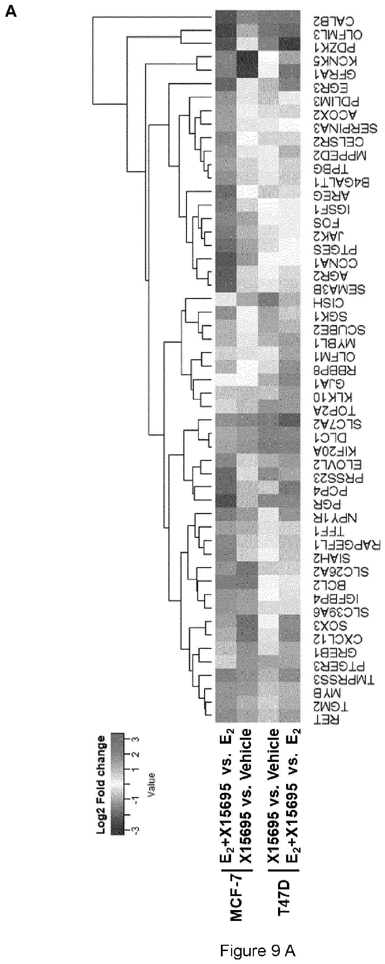

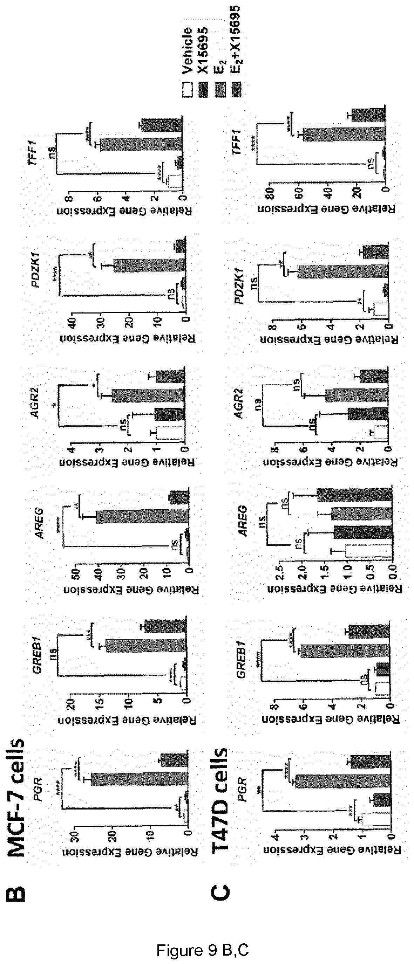

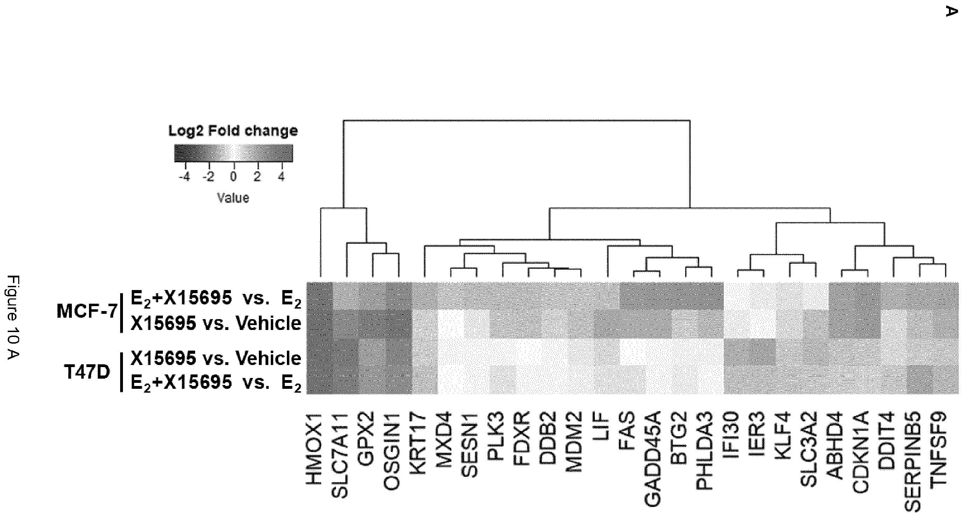

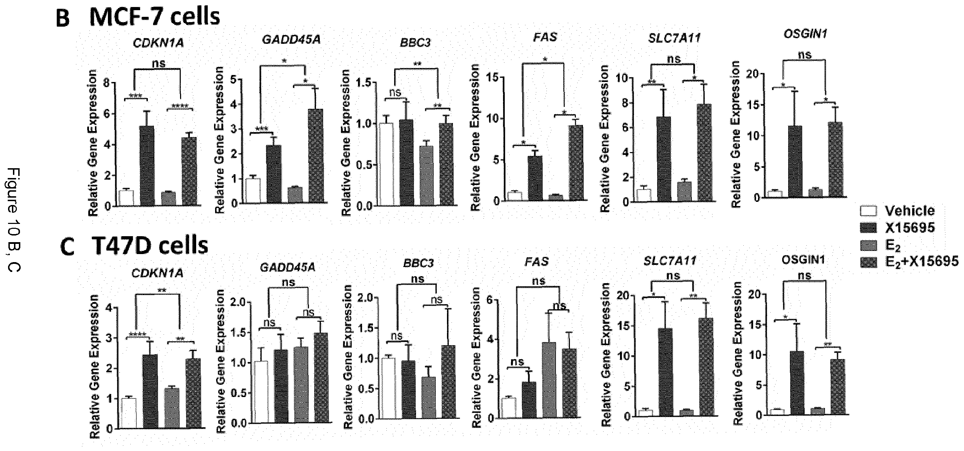

To investigate the pathways associated with the changes in gene expression brought about by X15695, Gene Set Enrichment Analysis (GSEA) was performed using the Hallmarks gene sets from the Molecular Signatures Database (MSigDB). Two of the most regulated pathways by X15695 in the presence of E₂ in MCF-7 cells were the ERα and p53 signaling pathways. In the absence of E₂, the p53 signaling pathway was also identified as the most regulated pathway (Fig. 7 A, B; Fig. 8 A, B). Among other signatures, the ERα and the p53 pathways were also the most significantly regulated pathways by X15695 in the T47D cells (Fig. 8 C, D). Therefore, a heatmap was generated to compare E₂ response genes in MCF-7 and T47D cells and this showed a very strong overlap in downregulation of expression of ERα target genes in the two cell lines (Fig. 9 A). Quantitative RT-PCR carried out with a select number of ERα response genes in the two cells showed indeed downregulation of E₂-mediated gene expression by X15695 (Fig. 9 B, C). On the contrary, the p53 pathway was upregulated by X15695 in the two cell lines as shown in the heatmap but more strongly in the MCF-7 cells compared to the T47D cells (Fig. 10 A). X15695-mediated upregulation of expression of p53 target genes in the absence and presence of E₂ was also confirmed in the qRT-PCR analyses with the results in the MCF-7 cells somewhat more prominent than in the T47D cells (Fig. 10 B, C).

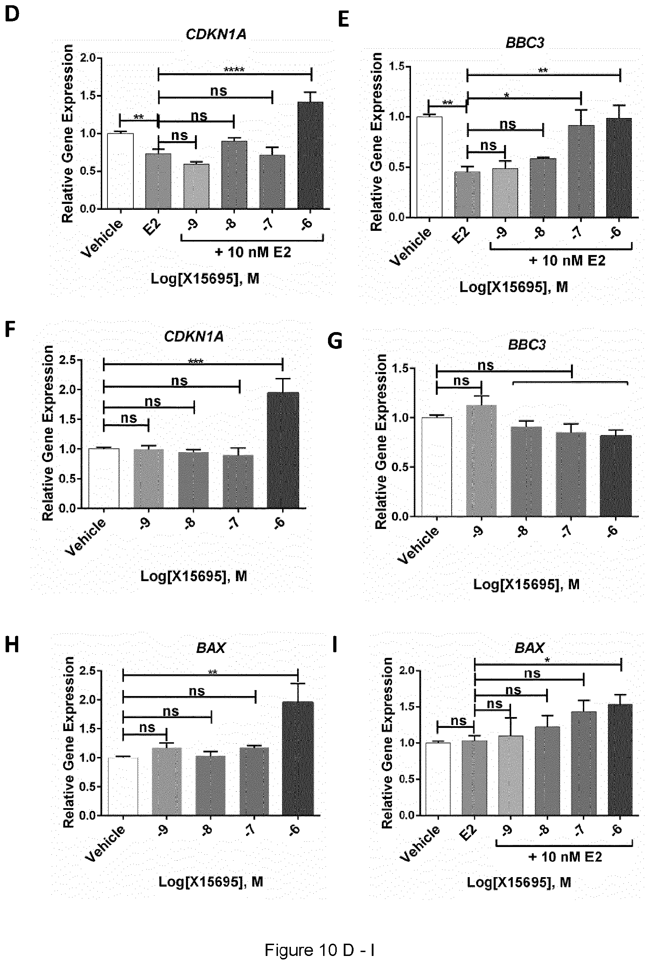

It was detected that X15695 did not only antagonize the positive regulation of gene expression by E₂, but negative regulation was also antagonized. For example, in MCF-7 cells E₂-mediated downregulation of expression of *CDKN1A* and *BBC3* was dose-dependently antagonized by X15695 (Fig. 10 D, E). Additionally, the expression of *CDKN1A* but not *BBC3* was upregulated by X15695 in the absence of E₂ (Fig. 10 F, G), making *CDKN1A* a gene under dual control mechanisms by E₂ and X15695. In contrast, the expression of the p53 downstream target gene *BAX,* was not regulated by E₂ but was transcriptionally enhanced by X15695 (Fig. 10 H, I). Combined, these findings identify X15695 as a compound that targets both the ERα and p53 signaling pathways and can therefore control the actions of the p53-ER regulatory loop described in breast cancers.

### Example 4:

### Mechanism of downregulation of ERα and upregulation of p53

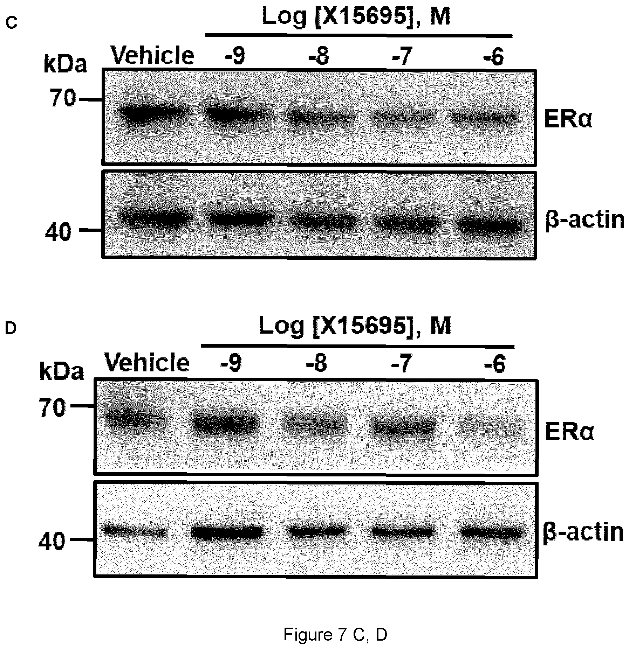

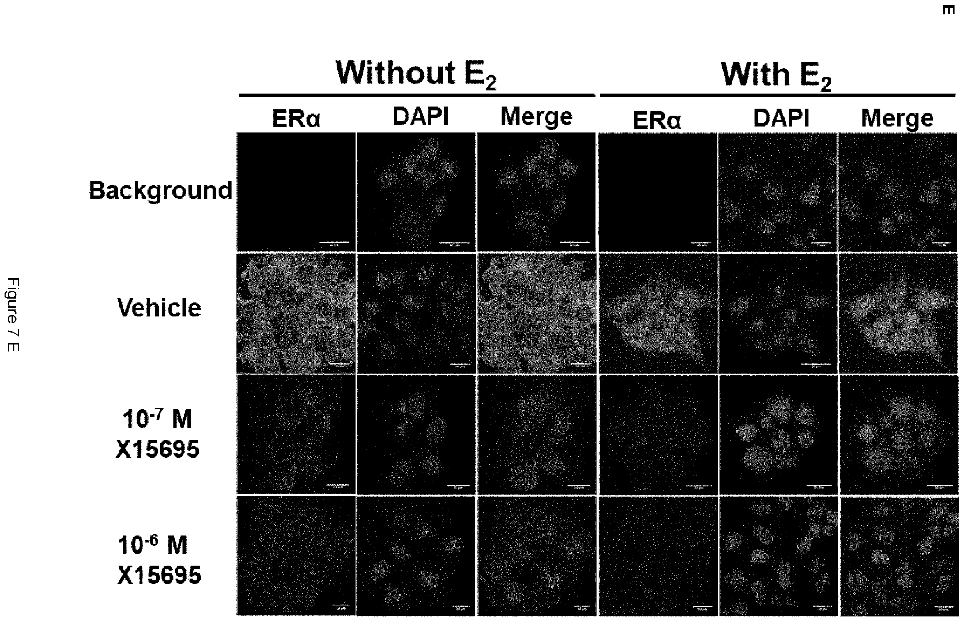

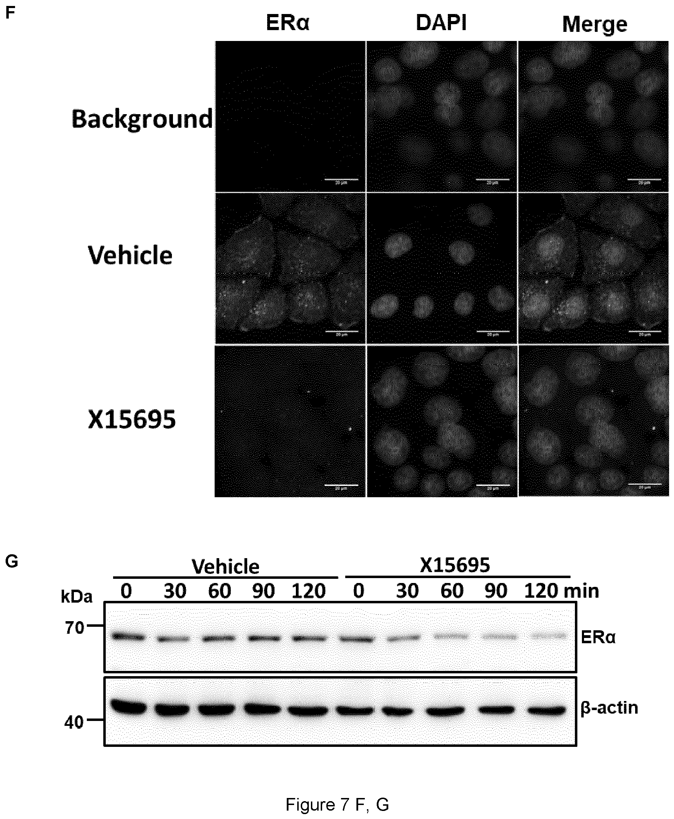

The possible mechanisms of X15695-mediated regulation of ERα and p53 signaling pathways in the breast cancer cells was investigated. In immunoblotting studies, X15695 dose-dependently decreased ERα level in both cell lines (Fig. 7 C, D). In immunofluorescence experiments in MCF7 cells, ERα staining in the absence and presence of E₂ was strongly reduced by X15695 treatment (100 nM and 1 µM) in the cytoplasm and in the nucleus (Fig. 7 E). A similar downregulation in ERα level was observed in immunofluorescence experiment in T47D cells (Fig. 7 F), albeit in full medium in the absence of added hormone.

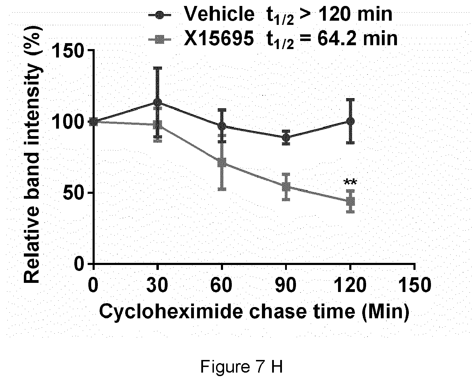

To determine how X15695 downregulates ERα level, MCF-7 cells were treated with cycloheximide that inhibits *de novo* protein synthesis and the degradation kinetics of the steady state population of the receptor was followed. It was found out that while ERα is relatively stable (> 120 min) in the absence of X15695, its half-life was reduced to about 60 min in the presence of X15695 (Fig. 7 G, H) suggesting destabilization of ERα by X15695.

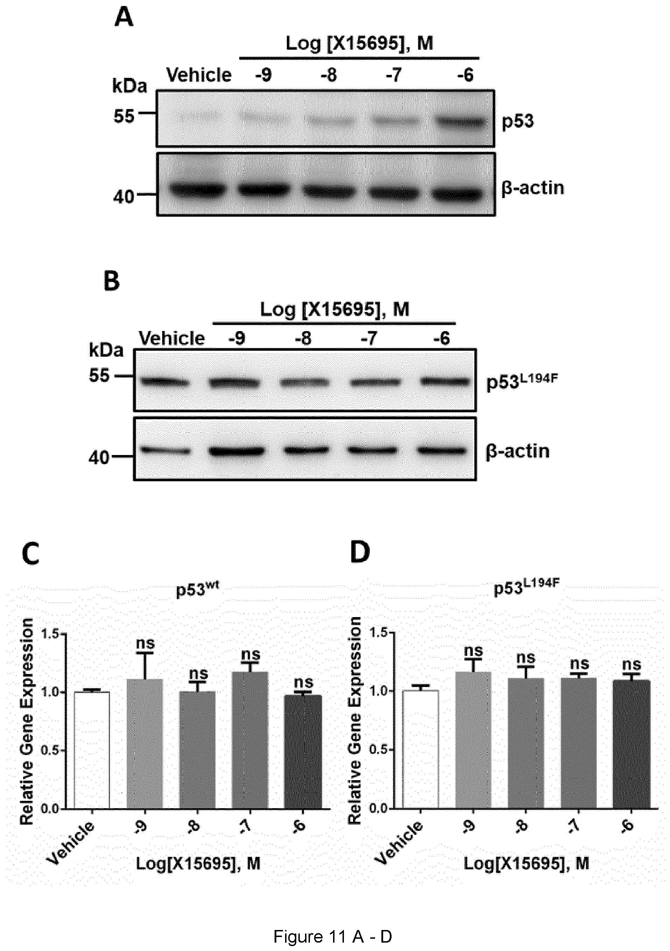

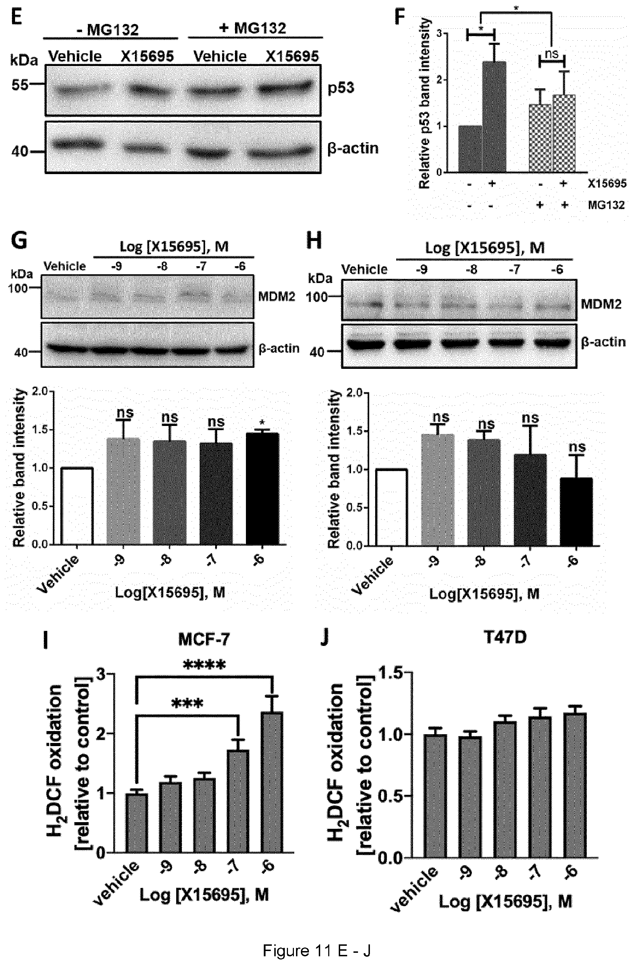

In contrast to decreasing ERα levels in both MCF-7 and T47D cells, X15695 differentially affected the level of p53 in the two cell lines. In Western blot experiments, p53 level was significantly increased by X15695 in MCF-7 but not in T47D cells (Fig. 11 A, B). Note that MCF-7 cells express a wild-type p53 while T47D cells, express a mutated p53 (L194F) indicating that they possibly undergo different regulatory processes. The upregulation of p53 expression in the MCF-7 cells did not occur at the RNA level as demonstrated in qRT-PCR experiments (Fig. 11 C, D), suggesting a regulation at the protein level. As protein turnover is a dynamic process controlled by the rate of protein synthesis and degradation, inhibition of protein degradation should provide information on the contribution of the latter process to the accumulation of p53. X15695 treatment in the absence of the proteasomal inhibitor MG132 led to an accumulation of p53 in the MCF-7 cells but in the presence of MG132 (Fig. 11 E), the basal level of p53 was increased and the upregulation by X15695 was abolished. A quantification of the effect of the MG132 and X15695 on p53 level presented in Figure 11 F shows that X15695 functions by inhibiting proteasomal degradation of p53. In the case of T47D cells, as the proteasomal degradation pathway is reportedly non-functional, mutant p53^{L194F} accumulated in the noninduced state and was no longer modulated by X15695 (Fig. 11 B).

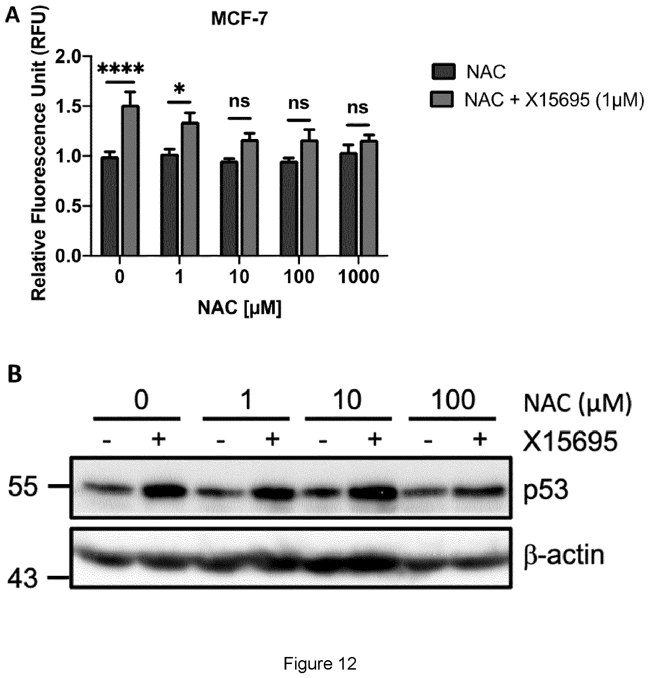

To further understand the mechanism leading to p53 accumulation, the expression level of MDM2, an E3 ubiquitin ligase that directs p53 degradation by the proteasome machinery, was analyzed. Immunoblot analysis showed that in MCF-7 cells, X15695 slightly upregulated MDM2 protein level (Fig. 11 G) rather than downregulating it to account of the negative effect of X15695 on p53 degradation. This suggests that X15695 action on p53 is independent of MDM2. In T47D cells, neither MDM2 nor p53 level was affected by X15695 treatment (Fig. 11 H). An alternative way for upregulation of p53 is through reactive oxygen species (ROS). This target is among the top processes in the GSEA analysis of both MCF-7 and T47D cells (Fig. 8). Therefore, ROS production was measured in MCF-7 and T47D cells using the fluorescent probe 2',7'-dichlorofluorescein diacetate (H₂DCF-DA). In MCF-7 but not in T47D cells, a significant dose-dependent production of ROS ensured after treatment of the cells with X15695 (Fig. 11 I, J). To show that ROS contributes to upregulation of p53, the ROS scavenger N-acetyl-L-cysteine (NAC) was used to inhibit the X15695-mediated increase in ROS level and the X15695-mediated upregulation of p53 level (Fig. 12 A, B).

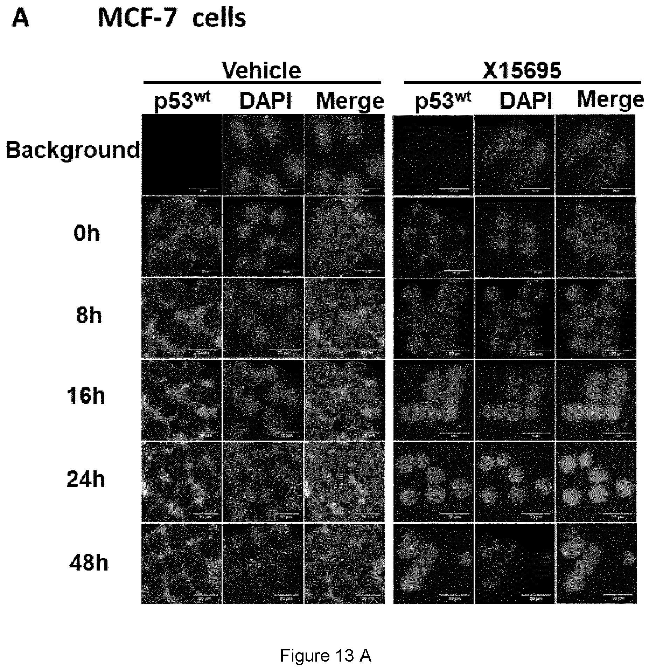

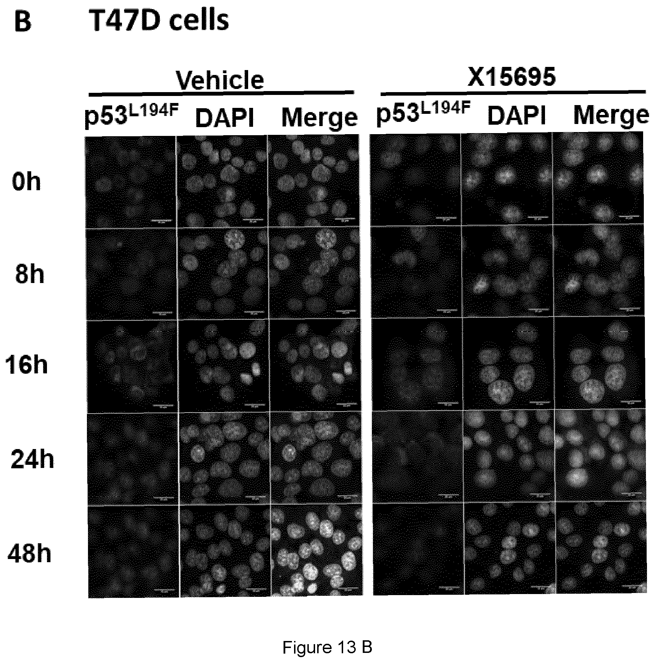

X15695 did not only influence the level of p53 but also regulated its cellular localization. In immunofluorescence studies, p53 in MCF-7 cells is mainly cytoplasmic but accumulated in the nucleus as early as 8 h after X15695 treatment (Fig. 13 A), while in T47D cells, p53 was already nuclear and its cellular localization was not further altered by X15695 treatment (Fig. 13 B).

### Example 5:

### X15695 disrupts p53-mortalin interaction

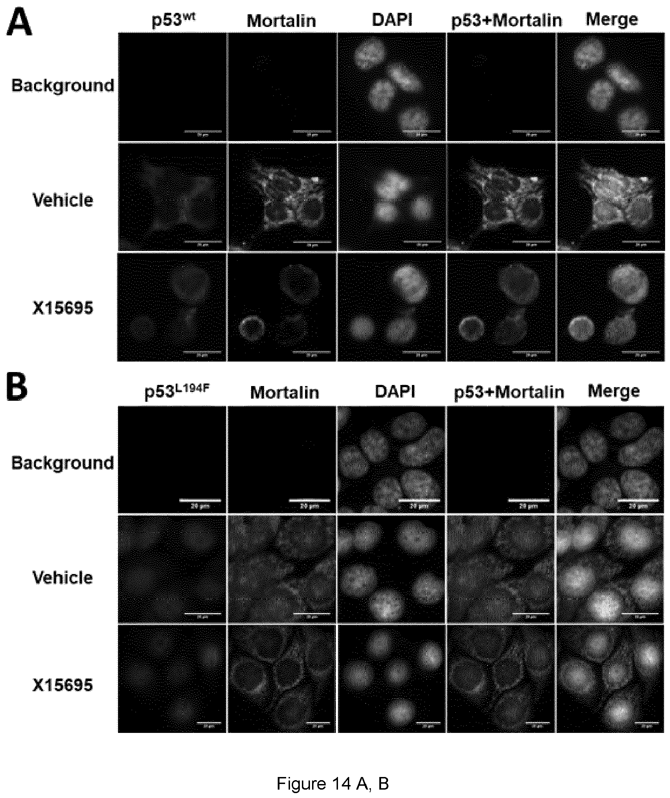

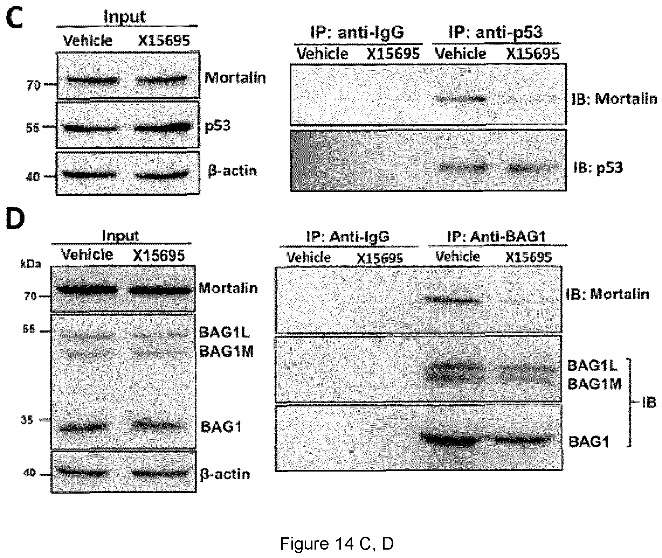

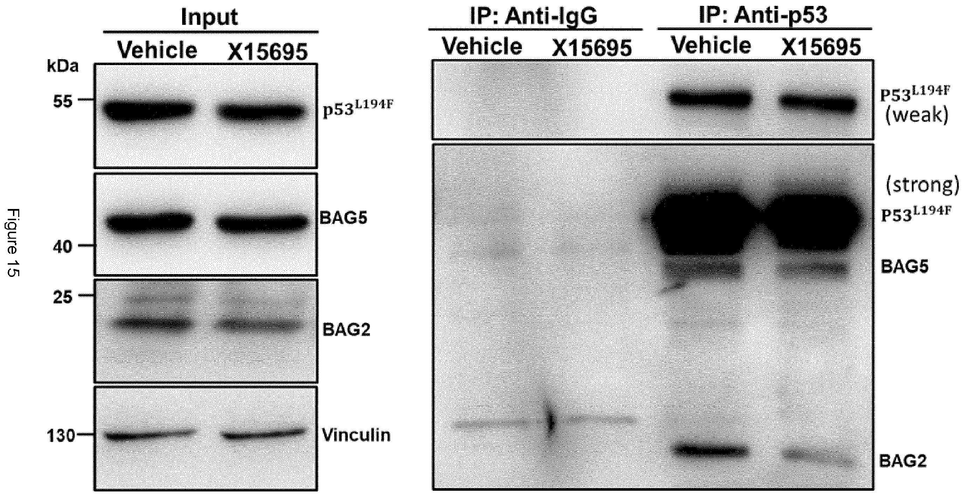

The cytoplasmic localization of wild-type p53 in tumor cells is enabled by sequestration by GRP75 (also known as mortalin), a member of the HSP70 molecular chaperone family. In immunofluorescence experiments in MCF-7 cells, this cytoplasmic colocalization of p53 and mortalin was confirmed but was altered by treatment with X15695. In the presence of X15695, p53 was translocated into the nucleus while mortalin assumed a perinuclear localization (Fig. 14 A). In contrast, p53L194F was nuclear in T47D cells and its cellular localization was not altered by X15695 treatment (Fig. 14 B). Coimmunoprecipitation experiments showed that in MCF-7 cells, p53 and mortalin were in a complex in the absence of X15695 but treatment with X15695 decreased this interaction (Fig. 14 C), indicating a disruption of the complex by X15695 to account for the nuclear translocation of p53. This regulatory action of X15695 maybe indirect since in coimmunoprecipitation experiments in MCF-7 cells, BAG1 (a binding partner of mortalin) was found in a complex with mortalin and treatment with X15695 decreased this interaction (Fig. 14 D). Thus, X15695 possibly contributes indirectly to the disruption of the p53/mortalin complex by also displacing the BAG1/mortalin complex. Unlike MCF-7 that expresses wild-type p53 that resides in the cytoplasm, T47D expresses a mutant p53 that is mainly nuclear. This mutant p53 is reported to promote tumorigenesis through a gain-of-function (GOF) mechanism involving nuclear interaction with BAG2 and BAG5. In co-immunoprecipitation experiments it was shown that X15695 disrupts the interaction of BAG2 but not BAG5 with mutant p53 in the T47D cells (Fig. 15), pointing to a possible mechanism of how this compound could relieve the GOF action of mutant p53.

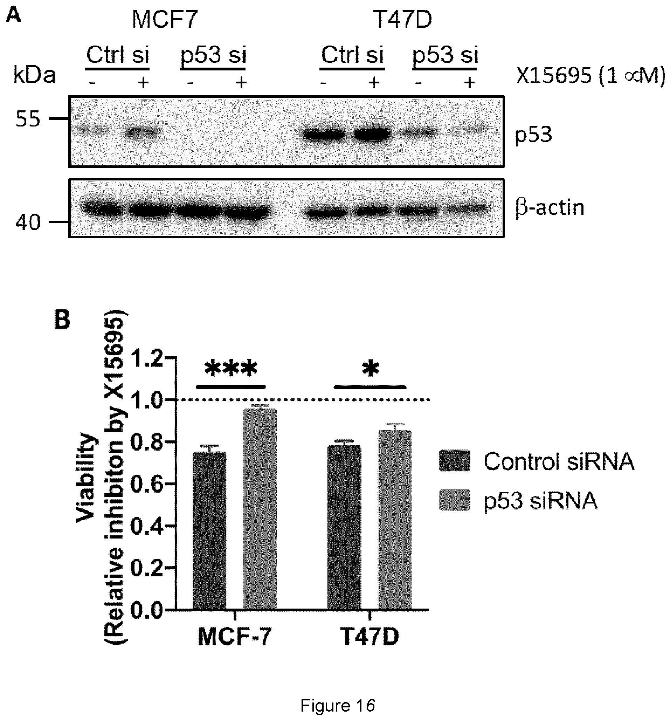

To further confirm a role of p53 in X15695 action, MCF-7 and T47D cells were transfected with control and a mixture of siRNAs against p53 and the inhibitory effect of X15695 on cell survival was analyzed. If p53 plays a role in the inhibitory action of X15695, then decrease in cell viability by this compound is expected to be attenuated by the knockdown of p53. The knockdown of p53 was almost complete in the MCF-7 cells but not in the T47D cells (Fig. 16 A). As a consequence, decreased cell survival mediated by X15695 was significantly attenuated in the p53 siRNA-transfected MCF-7 cells but somewhat compromised in the p53 knockdown T47D cells (Fig. 16 B). These studies demonstrate the involvement of p53 in the inhibitory action of X15695.

### Example 6:

### X15695 regulates cell cycle progression and apoptosis

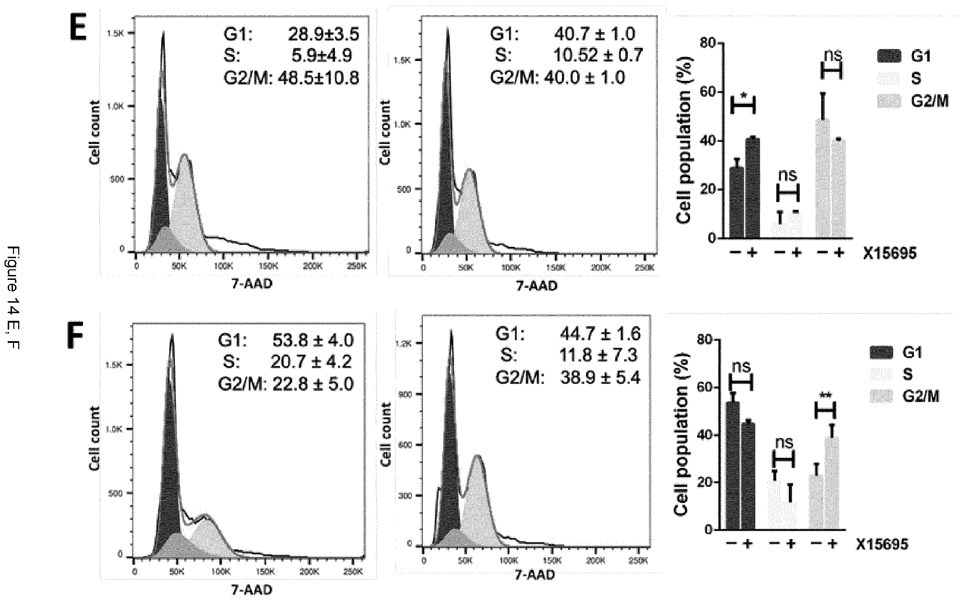

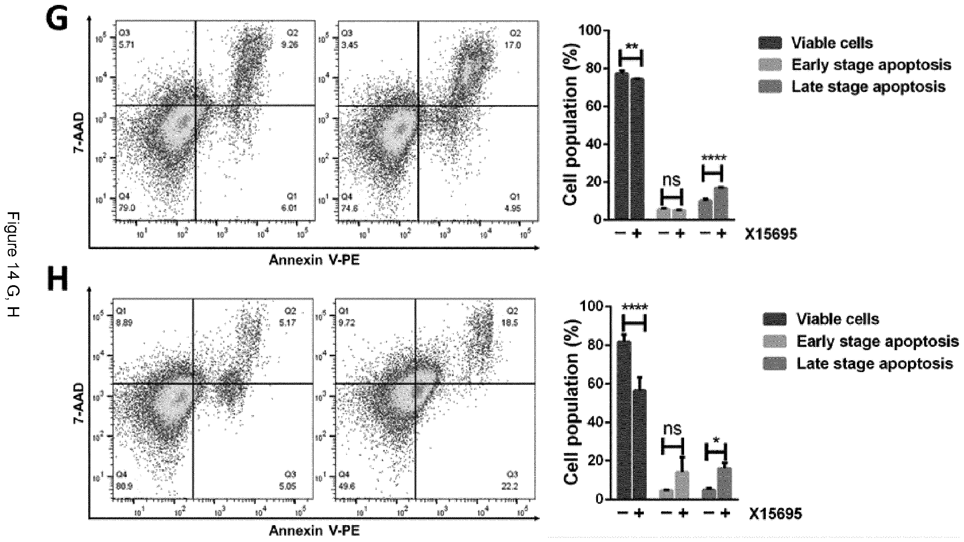

One of the functions of p53 in the nucleus is the regulation of its downstream targets such as p21, GADD45A, PUMA and BAX that control cell cycle progression and apoptosis. As genes encoding these proteins are significantly activated by X15695 in the breast cancer cells (Fig. 10 B, C), X15695 would be expected to induce cell cycle arrest and apoptosis. Cell cycle measurements determined by flow cytometry after treating MCF-7 and T47D cells with 7-Aminoactinomycin D (7-AAD) showed a G1/S phase arrest in the MCF-7 cells (Fig. 14 E) while in the T47D cells, a G2/M cell cycle arrest was observed (Fig. 14 F). Double staining with 7-AAD and Annexin V used for apoptosis evaluation revealed increased number of the cells in the late stage of apoptosis upon X15695 treatment in both MCF-7 and T47D cells (Fig. 14 G, H). Collectively these results show that X15695 inhibited ER⁺ breast cancer cells proliferation mainly through antagonism of ERα action and its regulation of p53 downstream effects.

### Example 7:

### X15695 regulation of tamoxifen-resistant MCF7 cells

Among the drugs approved for the treatment of ER+ breast cancers are tamoxifen and fulvestrant. Tamoxifen has a limitation in that it has some agonistic activities in addition to its antagonistic action, while fulvestrant, being an ERα degrader, is classified as a pure antagonist.

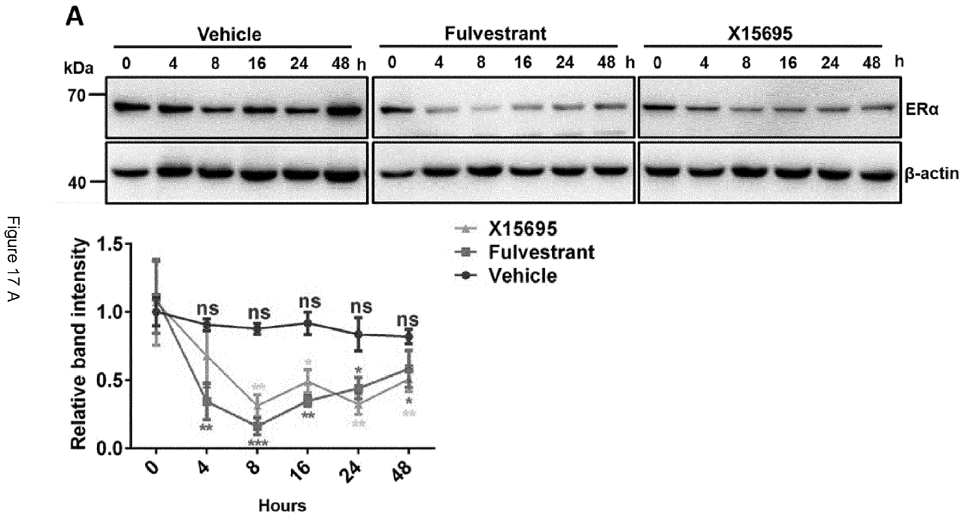

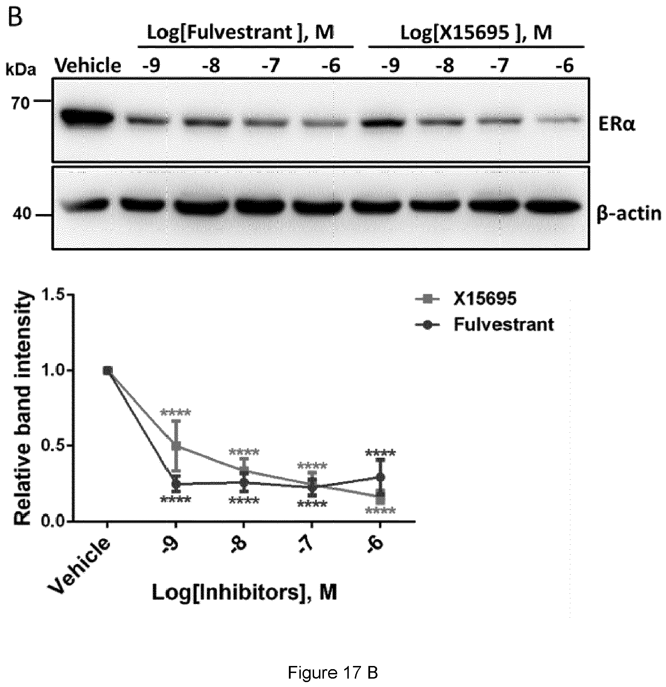

Since X15695, like fulvestrant, destabilizes ERα, the efficacies of the two compounds as ER antagonists were compared in a number of assays. Fulvestrant rapidly downregulated ERα level within 4 h to 8 h recovering gradually thereafter, while X15695 showed a rather slow but more sustained downregulation lasing over 24 to 48 h (Fig. 17 A). Both compounds at concentrations as low as 1 nM were sufficient to downregulate ERα in 8 h (Fig. 17 B).

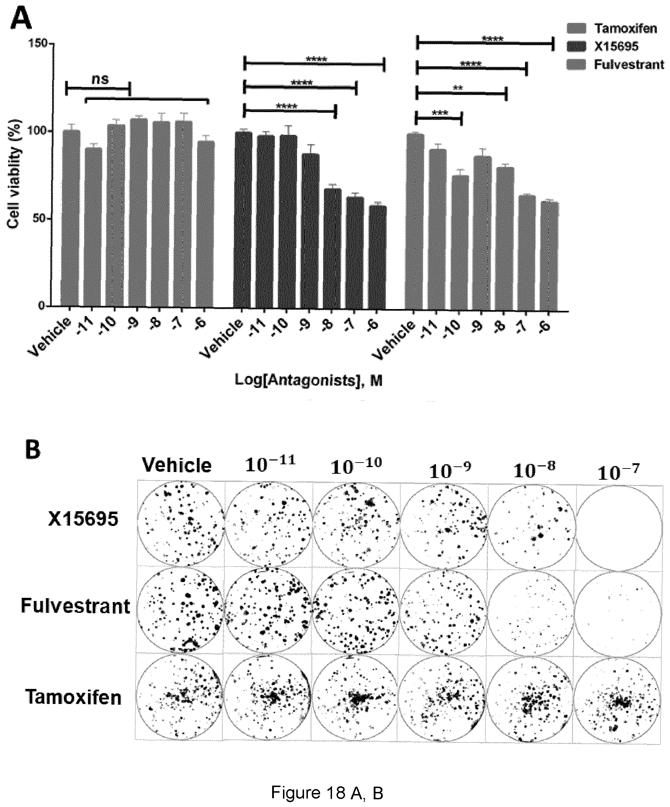

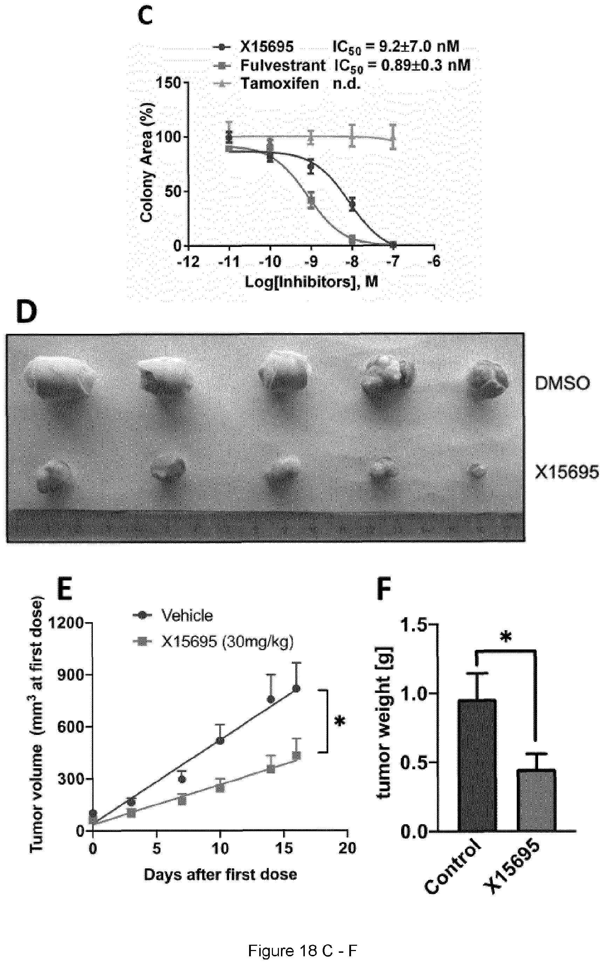

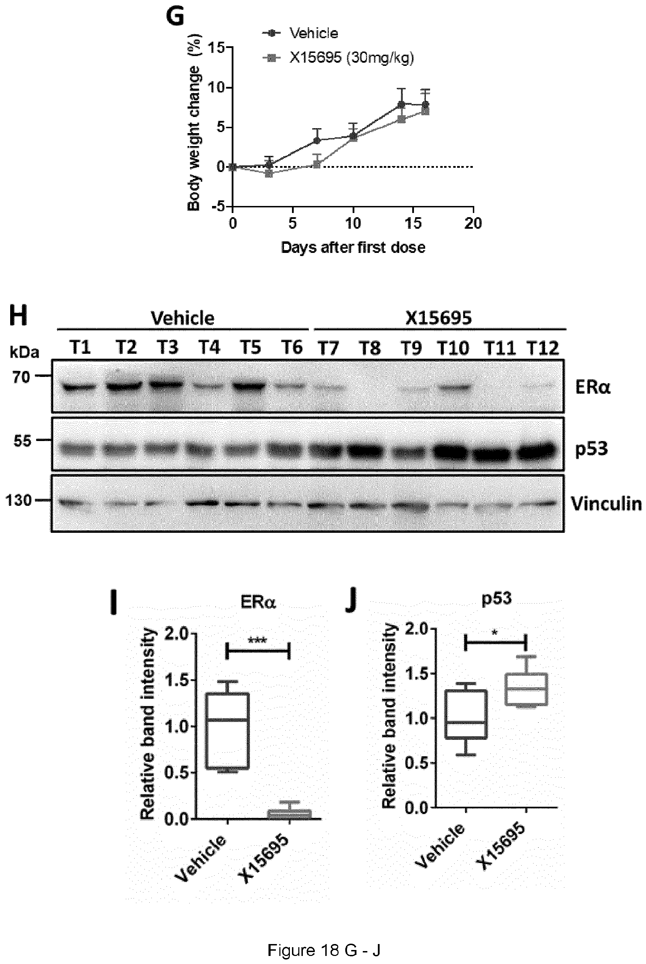

The two compounds were also compared based on their ability to inhibit the viability of TRMCF-7 cells (tamoxifen resistant MCF-7 cells). Here, X15695 and fulvestrant showed comparable activities as opposed to tamoxifen that was ineffective (Fig. 18 A). In clonal expansion experiments with TRMCF-7 cells, tamoxifen was again inactive while X15695 and fulvestrant were both active (Fig. 18 B, C) with fulvestrant slightly more efficacious than X15695 (Fig. 18 B, C).

In patients, fulvestrant is well tolerated but its unfavorable pharmacological properties warrant it to be administered intramuscularly. Several efforts are therefore being made to develop selective estrogen receptor degraders (SERDs) that can be administered orally. Therefore, the efficacy of X15695 in inhibiting tumor growth when applied orally was tested in a mouse xenograft tumor model. X15695 (30 mg/kg body weight) administered *per os* (P.O.) daily showed a significant decrease in tumor volume and weight within 2 weeks (Fig. 18 D-F). X15695 was well tolerated and did not cause any weight loss or other signs of host toxicity during the entire experiment (Fig. 18 G). Western blots on lysates of the tumors after the treatment period showed a significant decrease in ERα level and an increase in p53 expression in line with the results in the cell culture experiments (Fig. 18 H-J).

### Example 8:

### Regulation of AR⁺ prostate cancer cells by imidazopyridine derivatives

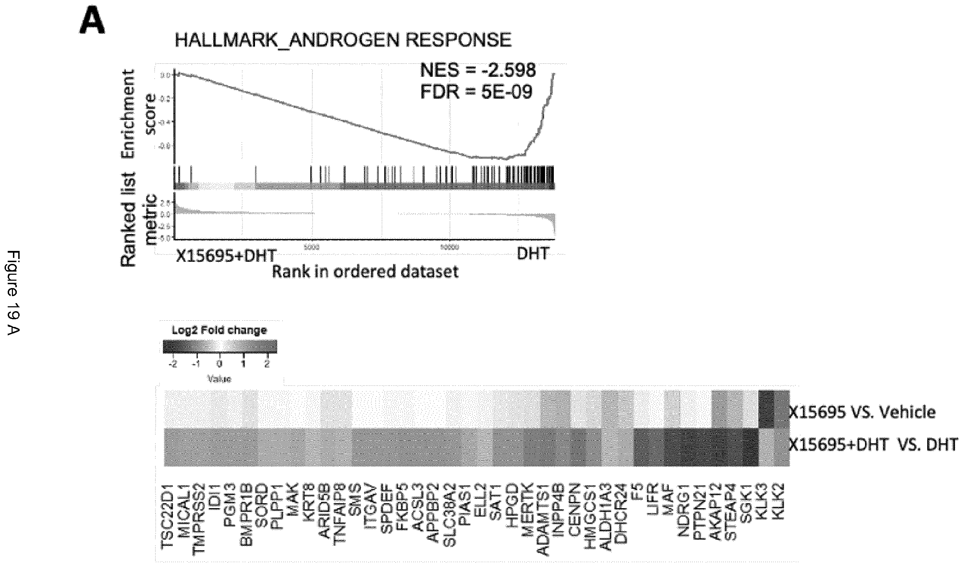

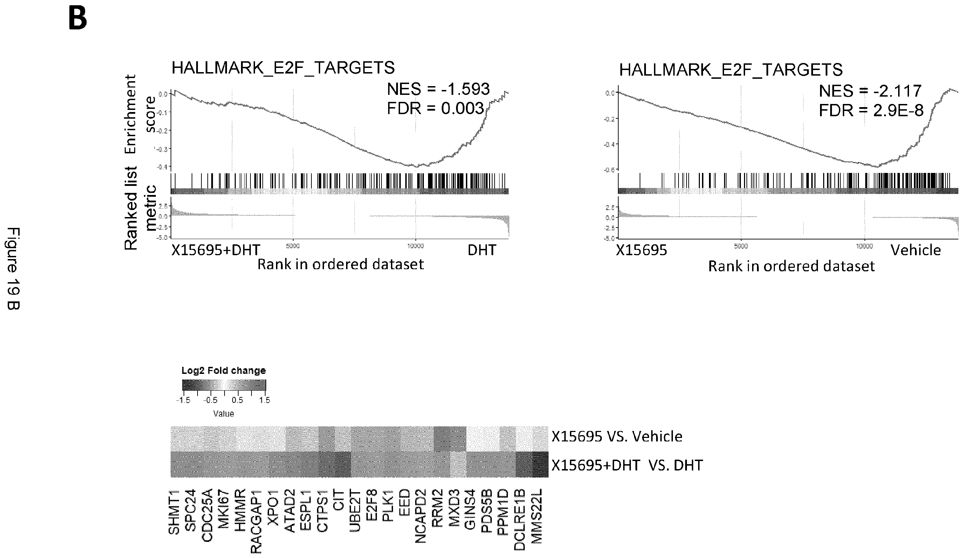

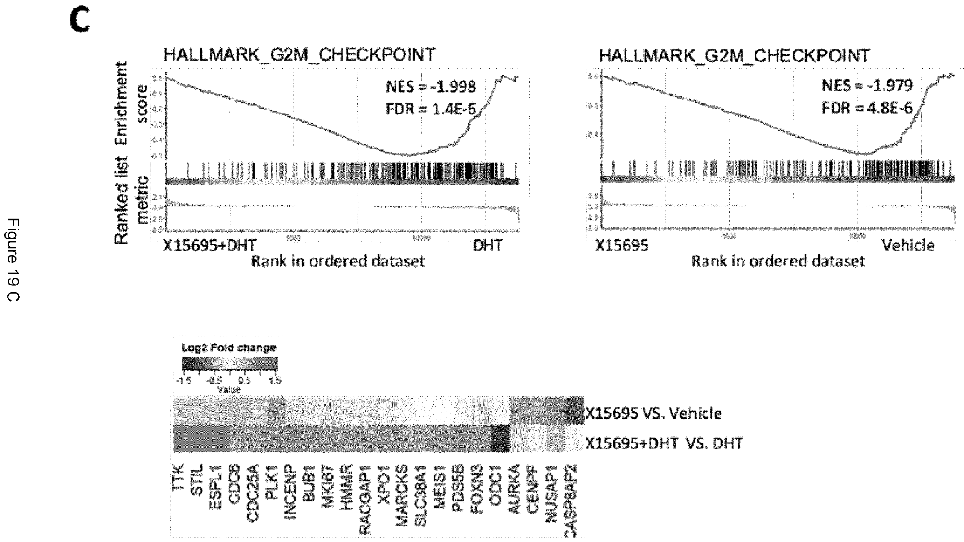

As the imidazopyridines that inhibited the clonal expansion of ER⁺ breast cancer cells also inhibited proliferation of AR⁺ prostate cancer (PCa) cells (Table 1), a possibility exists that these compounds function through a common anti-tumor mechanism. In transcriptomics experiments carried out with LNCaP cells treated with vehicle, X15695, DHT and DHT+X15695 showed an attenuation of androgen response. Specifically, the GSEA analyses showed that in the presence of DHT, androgen signaling pathway, G2/M cell cycle and E2F pathways were attenuated by X15695 treatment while in the absence of DHT, the expression of E2F targets and G2/M checkpoint genes was attenuated (Fig. 19 A-C).

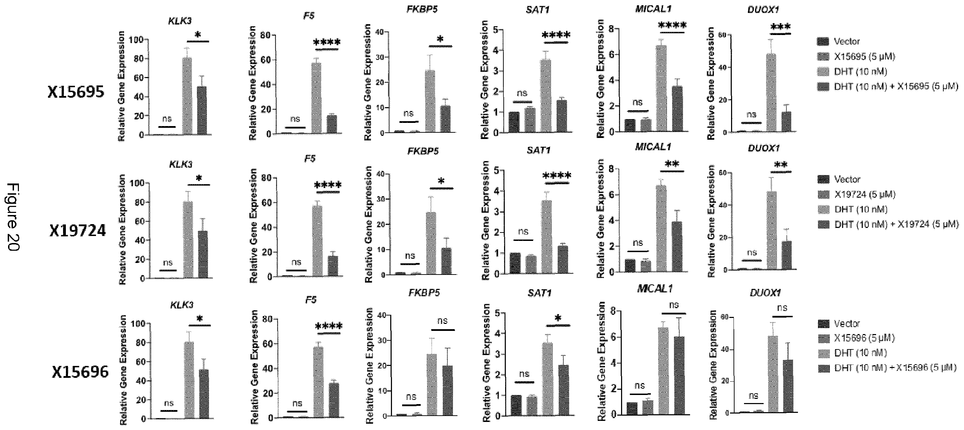

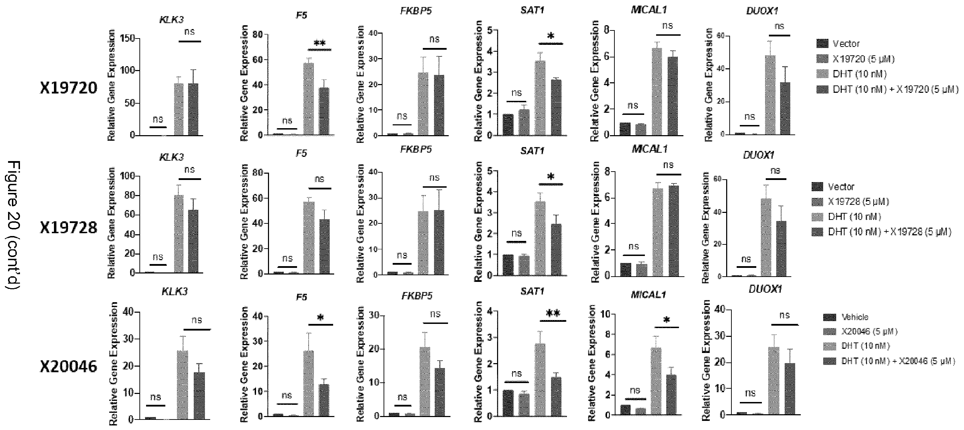

A select number of classical AR target genes (*KLK3, FKBP5, F5*) and androgen-induced ROS genes (*MICAL1, SAT1, DUOX1*) were used to determine the effect of the six selected imidazopyridines (Fig. 4) on AR response. These genes were also identified in the heatmap as AR regulated genes whose expression was attenuated by X15695 (Fig. 19 A). In qRT-PCR, androgen-induced AR target gene expression with 10 nM DHT was attenuated by a number of the imidazopyridines at ≥ 5 µM and X15695 was identified as the compound with the most inhibitory activity (Fig. 20). In studies on the upregulation of p53 expression by the selected imidazopyridines, no major differences were observed in the action of the different compounds in three different prostate cancer cell lines used for the study (Fig. 22 A). Therefore, the further study focused on X15695 to allow for a better comparison of the PCa with the breast cancer studies.

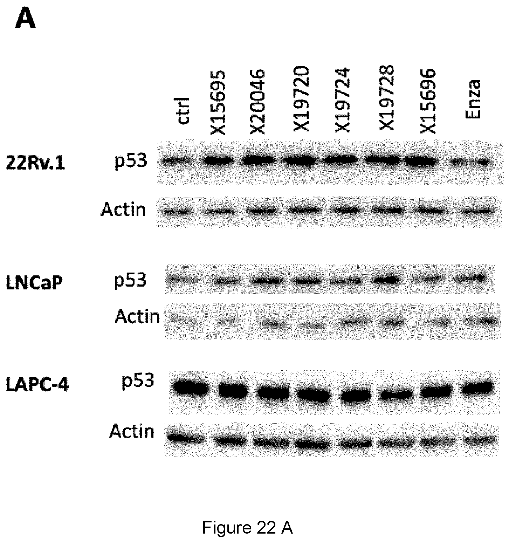

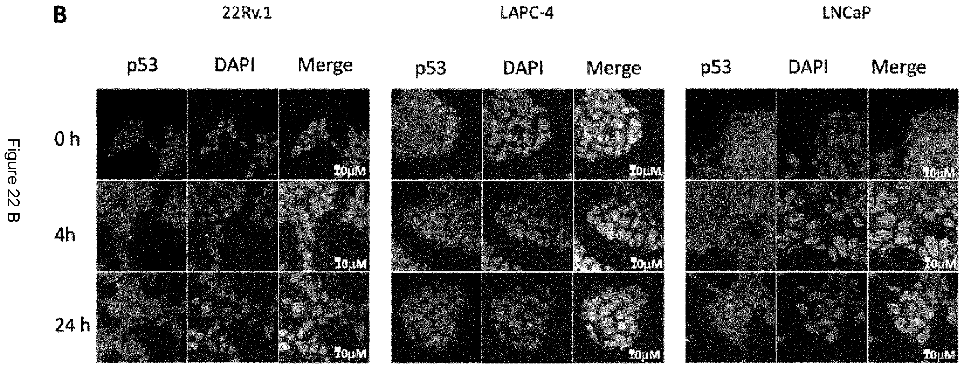

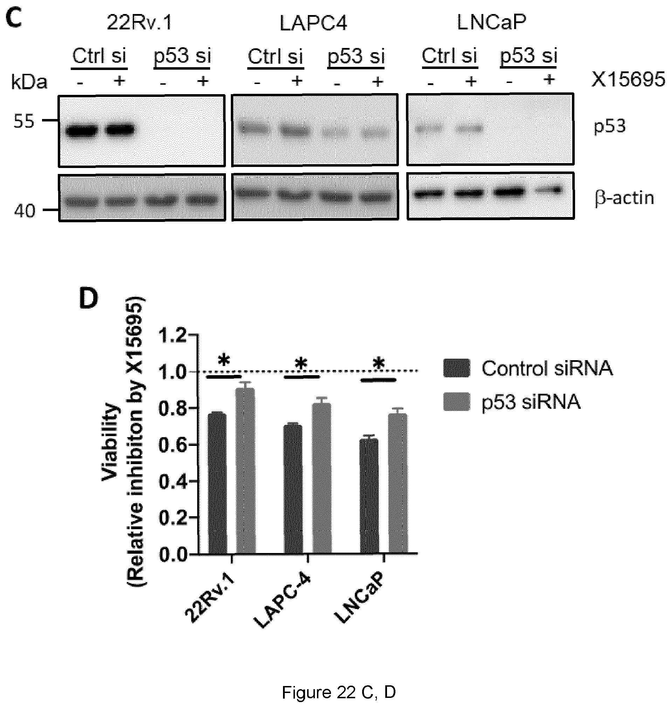

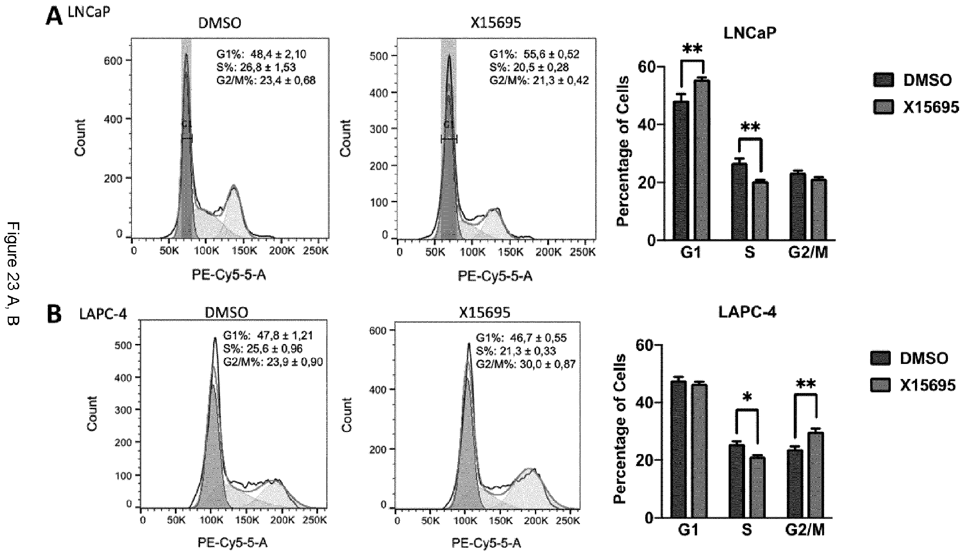

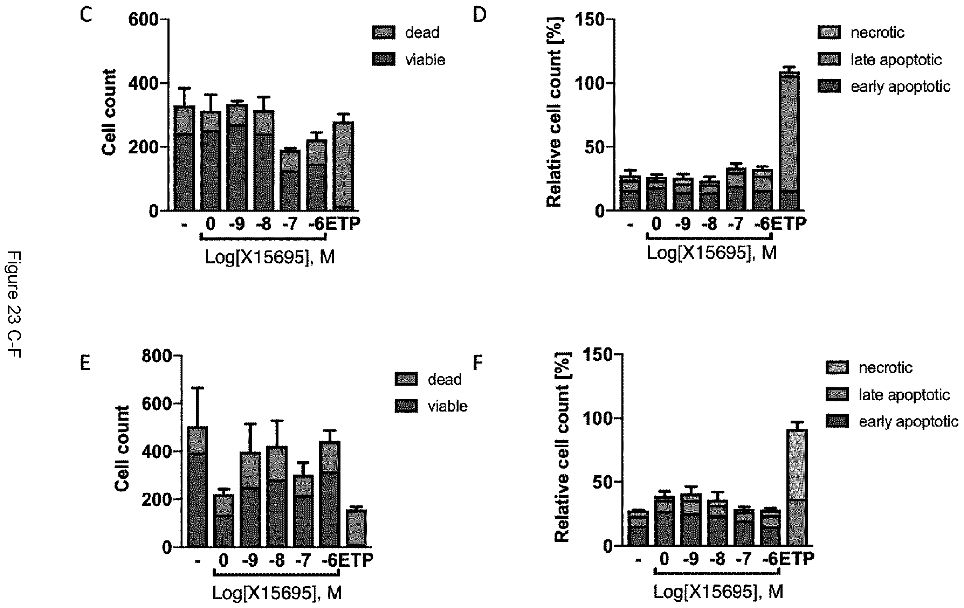

It was investigated whether X15695 caused nuclear translocation of p53 as was observed in the MCF-7 cells. In this context, it should be noted that the PCa cells have different p53 status: LNCaP cells express wt p53; 22Rv.1 cells express wtp53/mtp53^{Q331R} and LAPC-4 cells express mtp53^{R175H}. In immunofluorescence experiments, X15695 promoted a cytoplasmic/nuclear translocation of p53 in cells that express wild-type p53 (LNCaP and 22Rv.1) but not in LAPC-4 cells that express only mutp53. In the latter cell line, p53 was already nuclear and X15695 did not alter its cellular localization (Fig. 22 B). To determine whether p53 plays a role in the inhibitory action of X15695 in the PCa cells, the X15695-mediated decrease in viability of the three prostate cancer cells (22Rv.1, LNCaP and LAPC-4) was determined after knockdown of p53 by siRNA transfection (Fig. 22 C). Decreased viability observed after treatment of these cells with X15695 was significantly compromised in all three cell lines transfected with p53 siRNA (Fig. 22 D), indicating a contribution of p53 to the growth inhibitory action of X15695 in these cells. It is believed that X15695 reactivated wt and mutant p53 in these cells as was shown in the breast cancer cells. Therefore, flow cytometry analysis was performed to determine changes in the cell cycle following treatment of LNCaP and LAPC-4 cells with X15695. In the LNCaP cells, this treatment led to a G1/S phase arrest while in the LAPC-4 cells a G2/M arrest was observed (Fig. 23 A, B). No apoptotic effect of X15695 was found in either prostate cancer cell type (Fig. 23, C-F), indicating that the X15695-mediated inhibition clonal expansion in these cells may probably be due to a cell cycle arrest possibly senescence.

### Example 9:

### X15695 functions synergistically with chemotherapeutic drugs

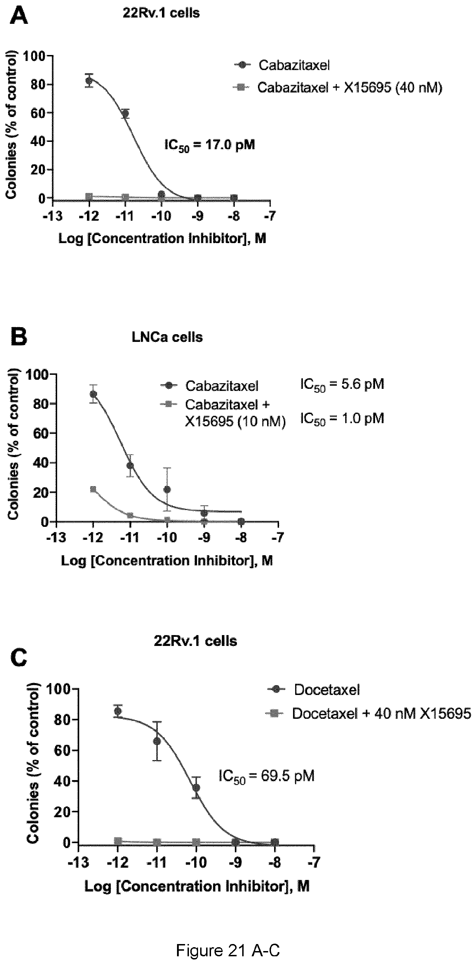

The ability of X15695 to trigger cell cycle block and stop cells from growing prompted the question whether it can be combined with docetaxel and cabazitaxel that induce cell death via microtubule depolymerization and stabilization. Treatment of LNCaP and 22Rv.1 prostate cancer cells with the above chemotherapeutic drugs significantly decreased clonal expansion of these cells in the nanomolar and picomolar range. However, a combination of the chemotherapeutic drugs and X15695 even more effectively decreased the inhibitory activity of these compounds demonstrating a synergistic mechanism of action (Fig. 21 A-C).

### Example 10:

### X15695 inhibits prostate tumor growth in a mouse xenograft model

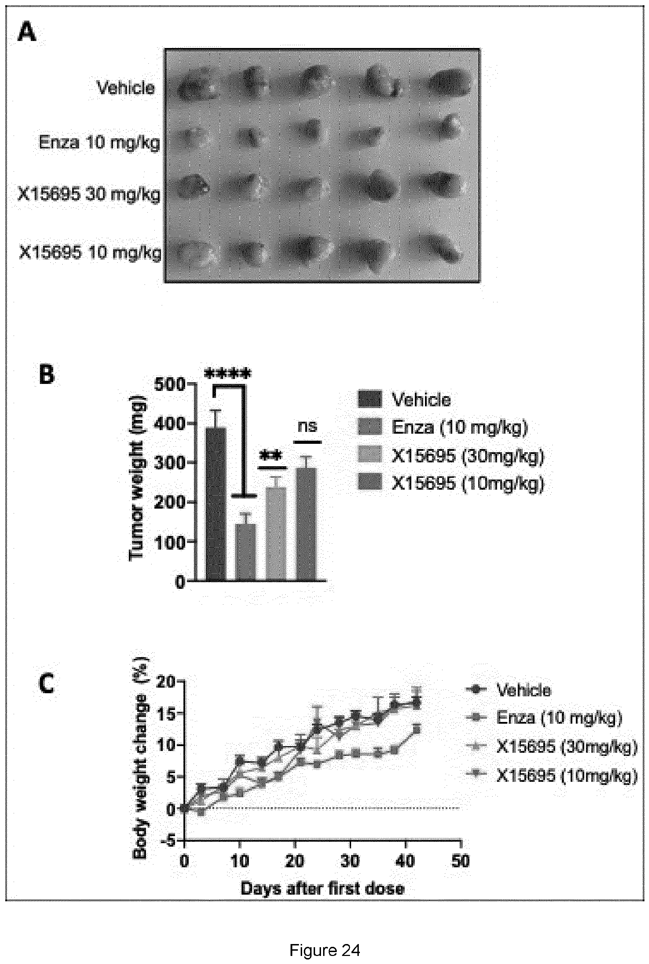

To determine whether the cell cycle block and the anti-androgen action of X15695 are sufficient to inhibit prostate tumor growth, mouse tumor xenograft experiments were carried out using LAPC-4 cells that express wild-type AR and are strictly dependent on androgens for proliferation. The action of X15695 (10 and 30 mg/kg) was compared with the classical antiandrogen enzalutamide (10 mg/kg) over 42 days. X15695 was shown to effectively inhibit tumor growth over vehicle upon oral administration (30 mg/kg/day), albeit less effectively as enzalutamide (10 mg/kg/day) (Fig. 24 A, B), indicating that X15695 is a weaker anti-tumor agent in this model compared to enzalutamide. As with the MCF-7 cell xenograft experiment, X15695 was well tolerated during the study and no sign of toxicity or weight loss was observed (Fig. 24 C).

### Example 11:

### X15695 is selective for ER⁺ breast cancer and endometrial cancer cells

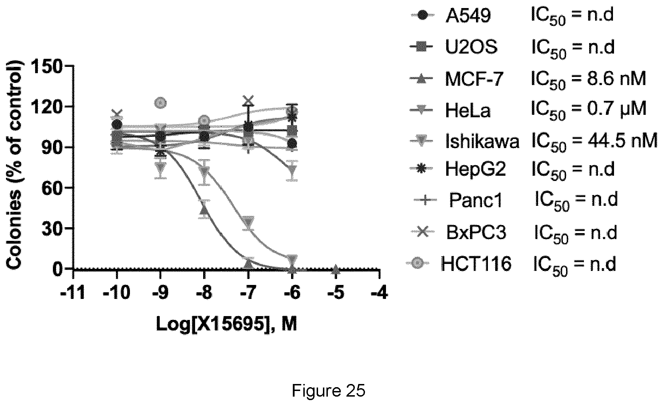

The negative action of X15695 on proliferation of AR⁺ prostate cancer in addition to ER⁺ breast cancer posed the question as to what other tumor cells will be inhibited by this compound. The effect of X15695 on the clonal expansion of several tumor cell lines consisting of cervical cancer, lung cancer, osteosarcoma, hepatoma, pancreatic cancer, colorectal carcinoma, endometrial cancer, and breast cancer (HeLa, A549, U2OS, HepG2, Panc1, BxPC3, HCT116, Ishikawa and MCf-7) was analyzed. While X15695 dose-dependently inhibited the clonal expansion of MCF-7 and Ishikawa cells, it did not have any effect on the other tumors cell lines (Fig. 25). The inhibitory effect of X15695 on Ishikawa and MCF-7 cells can most likely be attributed to the ERα positivity of these cells.

Collectively these results demonstrate that X15695 inhibits proliferation of breast cancer cells and PCa cells but its effect on the degradation of ERα combined with the reactivation of p53 makes it a superior inhibitor of ER⁺ cancer cells than the AR+ PCa cells. These effects combined with its lack of action in inhibiting proliferation of other cancer cells warrants X15695 to be classified as an oral selective estrogen receptor degrader (SERD).

### Discussion:

In this study a compound with an imidazopyridine scaffold X15695 was shown to strongly downregulate ERα activity and to a lower extent AR action and to reactivate wild-type and mutant p53. As a consequence, X15695 inhibited proliferation of ER⁺ breast cancer and AR⁺ prostate cancer cells *in vitro* and *in vivo* in xenograft models. However, in the *in vivo* studies X15695 was more efficacious in inhibiting breast cancer than prostate cancer cell proliferation. X15695 action was selective. Apart from its inhibition of proliferation of an endometrial cell line which is also ER+, it did not inhibit the proliferation of steroid receptor negative breast or prostate cancer cells or other tumor cell lines.

Imidazopyridine based compounds to which X15695 belongs have gained significant attention in medicinal chemistry due to their frequent occurrence in a large number of marketed drug formulations and drug candidates. These compounds have a wide variety of biological and pharmacological activities such as anti-mycobacterial, anti-diabetic, anti-viral, and anti-cancer activities. In the field of anticancer drugs, a library of amide derivatives of imidazo-pyridine has been synthesized and shown to be highly potent in inhibiting the proliferation of breast (MCF-7, MDA MB-231), lung (A549), and prostate (DU145) cancer cell lines. Recently, imidazopyridine compounds that inhibit phosphoinositide-3-kinase/Akt (PI3K/Akt) and the proliferation of AR⁺ and AR⁻castration-resistant prostate cancer cells have been described. However, the high concentrations of these compounds (up to 10 µM) required for the inhibition of CRPC proliferation question their on-target mode of action. These compounds also differ structurally from the imidazopyridines described in this work and their inhibitory profile is less selective compared to the compounds described here.

Several *in vitro* experimental observations have shown that the anticancer effects of the previously described imidazopyridine compounds are derived mainly from their inhibitory action on six main molecular targets: PI3K/Akt, centromere-associated protein E(CENP-E), insulin-like growth factor-1 receptor (IGF-1R), cyclin-dependent kinases (CDKs), tubulin polymerization, and hepatocyte growth factor receptor (HGFR). So far, other than our present study, steroid receptor action or p53 signaling have not been proven to be molecular targets of imidazopyridines.

The present studies also show that the imidazopyridines described in this work may function as orthosteric inhibitors by interfering directly with protein-protein interfaces, and thereby disrupting binding of cellular factors. For example, X15695 disrupts the interaction of cochaperone BAG1 with mortalin as well as the interaction of mortalin with wild-type p53 in the cytoplasm of MCF-7 cells. Disruption of the cytoplasmic retention of p53 leads to the nuclear translocation of p53 and the activation of the cell cycle regulator p21 and the apoptotic action of p53. Reactivation of p53 has remained a challenge in drug discovery programs for anticancer therapies. Several small molecule inhibitors are reported to disrupt mortalin-p53 to reactivate p53. Among them is Mortaparib^{plus} that inhibits the proliferation of MCF-7 cells. However, Mortaparib^{plus} reactivates wild-type p53 but not mutant p53 in T47D cells. Other compounds that reactivate mutant p53 have been reported. The most promising is PRIMA-1 and its methyl analog Eprenetapopt (APR-246). APR-246 is a first-in-class small molecule that restored wild-type p53 functions in TP53-mutant cells and is currently in phase II clinical trials. Unlike Mortaparib^{plus} and APR-246, it has been shown herein that X15695 reactivates both wild-type and mutp53. With mutant p53, it was shown that it disrupts its interaction with the cochaperone BAG2 and thereby abrogates the GOF action of mutp53.

With the X15695-mediated disruption of BAG2/mutp53 interaction reported herein, the proliferation of a large number of tumor cells with mutant p53 would be expected to be inhibited by X15695. This was evidently not the case herein as X15695 did not significantly alter the proliferation of ER negative breast cancer cell line MDA-MB231 that expresses mutant p53 R280K or AR⁻ prostate tumor cell line DU145 that expresses mutant p53 (P223L/V274F). These findings suggest that other regulatory factors, in addition to p53, are required for X15695 to exert its maximum inhibitory action. These other regulatory factors could be the steroid receptors.

It was shown that X15695 inhibits proliferation of tumor cells that express the ER and AR, but its effect is more profound for breast compared to prostate cancer cells. This selectivity of action may stem from the fact that the oncogenic action of ERα in the breast cancer cells may be attenuated by an additional regulatory action involving X15695. A careful analysis of the volcano plots of RNA sequencing data reported in this work shows the upregulated expression of several AhR target genes such as Cyp1A1, Cyp1A2, Cyp1B1 and ALDH3A1 upon treatment of breast cancer cells with X15695. It is therefore likely that the AhR is a target of X15695 and can contribute to the degradation of the ERα. through a previously reported AhR- ERα signaling pathway.

One of the important findings of the present invention is that X15695 downregulates ERα levels which puts it in the class of ER degraders to which the clinically approved antiestrogen fulvestrant belongs. However, unlike fulvestrant that requires intramuscular injection, X15695, when given orally, reduces ERα levels in two weeks in a xenograft tumor model. A further advantage of X15695 is that it induces cell cycle arrest and apoptosis to enhance its antitumor action in breast cancer cells without the need for combination with other drugs. In contrast, to improve the anti-cancer action of fulvestrant, it is recommended to have it used together with other targeted therapies such as CDK4/CDK6 inhibitors (e.g. Palbociclib) that promote cell cycle block at the G1/S phase.

Combined these differences in the action of X15695 compared to fulvestrant present X15695 as a selective orally available ERα degrader that warrants its further development as an ER⁺ breast cancer therapeutic.

## Claims

1. A compound for use in the treatment of cancer in a subject, wherein the cancer is selected from the group consisting of breast cancer, prostate cancer, and endometrial cancer, and wherein said compound is a compound according to Formula (I): 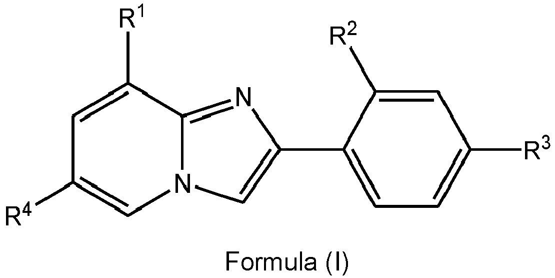 wherein
R¹ is H or Cl,
R² is H or F,
R³ is H or F, and
R⁴ is CH₃ or CF₃.

2. The compound for use of claim 1, wherein the compound according to Formula (I) is a compound, selected from the group consisting of the following compounds X15695, X15696 (=X19166), X19720, X19724, X19728, and X20046:

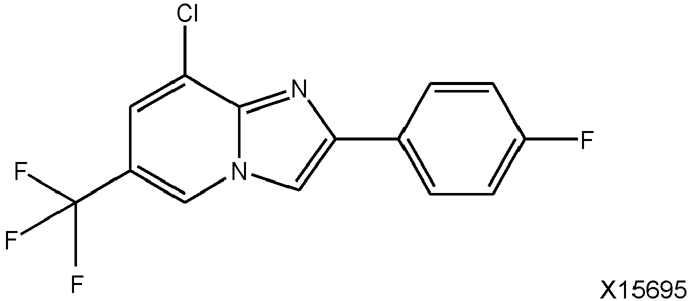

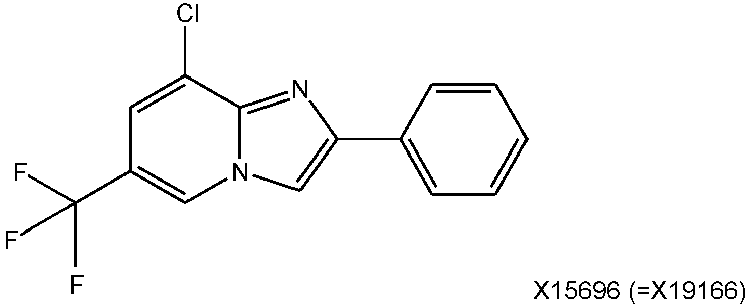

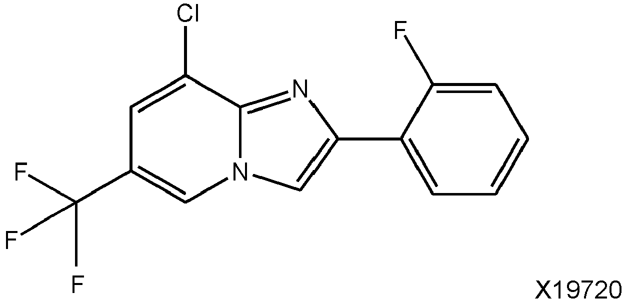

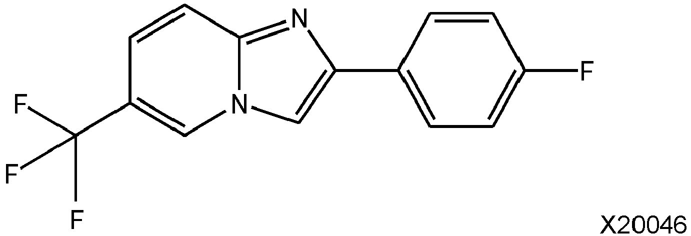

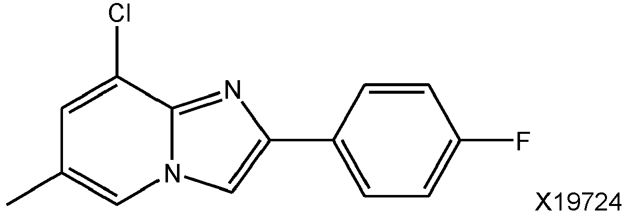

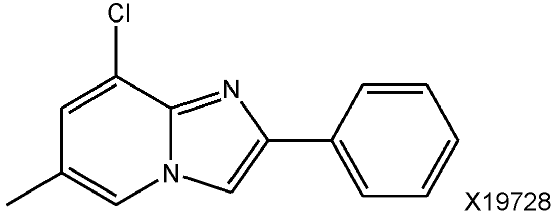

3. The compound for use of claim 1 or claim 2, wherein the compound according to Formula (I) is compound X15695:

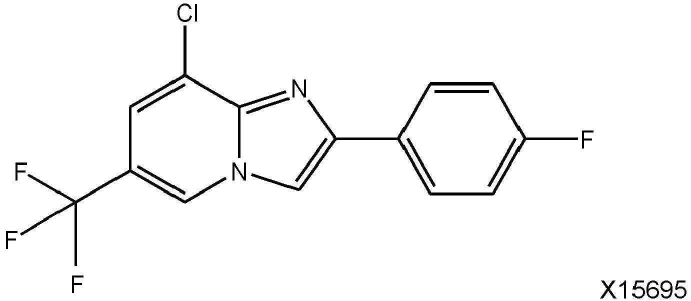

4. The compound for use of any one of claims 1 to 3, wherein the cancer is breast cancer.

5. The compound for use of claim 4, wherein the breast cancer is estrogen receptor-positive breast cancer.

6. The compound for use of any one of claims 1 to 3, wherein the cancer is prostate cancer.

7. The compound for use of claim 6, wherein the prostate cancer is androgen receptor-positive prostate cancer.

8. The compound for use of any one of claims 1 to 3, wherein the cancer is endometrial cancer.

9. The compound for use of claim 8, wherein the endometrial cancer is estrogen receptor-positive endometrial cancer.

10. The compound for use of any one of claims 5, 7, and 9, wherein the estrogen receptor is estrogen receptor α (ERα) and/or the androgen receptor is androgen receptor (AR).

11. The compound for use of any one of claims 1 to 10, wherein said compound is administered in combination with a chemotherapeutic drug.

12. The compound for use of claim 11, wherein the chemotherapeutic drug is selected from the group consisting of doxorubicin, epirubicin, paclitaxel, docetaxel, cabazitaxel, mitoxantrone, estramustine, and combinations thereof.

13. The compound for use of claim 11, wherein the chemotherapeutic drug is selected from the group consisting of docetaxel and cabazitaxel.

14. The compound for use of any one of claims 1 to 13, wherein the subject is a human subject.

15. The compound for use of any one of claims 1 to 14, wherein said compound is administered orally.
